# EUROPEAN PATENT SPECIFICATION

(11) **EP 3 146 899 B1**
(45) Date of publication and mention of the grant of the patent: **20.11.2019**
(21) Application number: 15796862.9
(22) Date of filing: 24.04.2015
(51) Int. Cl.: A61B 6/00, B22F 1/00, B22F 9/00, C09D 4/02, C09D 5/14, G06F 3/041, G01T 7/00, A01N 59/16

(54) **DEVICE, PROTECTING SHEET, AND ANTIBACTERIAL FILM**
VORRICHTUNG, SCHUTZFOLIE UND ANTIBAKTERIELLER FILM
DISPOSITIF, FEUILLE DE PROTECTION ET FILM ANTIBACTÉRIEN

(30) Priority: 22.05.2014 JP 2014106433; 15.07.2014 JP 2014145451; 30.09.2014 JP 2014201830
(43) Date of publication of application: 29.03.2017
(73) Proprietor: Fujifilm Corporation, Minato-ku Tokyo 106-8620 (JP)
(72) Inventor: NARIYUKI Fumito, Fujinomiya-shi Shizuoka 418-8666 (JP); SHIBATA Michihiro, Fujinomiya-shi Shizuoka 418-8666 (JP); NAGASAKI Hideo, Fujinomiya-shi Shizuoka 418-8666 (JP); OMAE Norihiro, Fujinomiya-shi Shizuoka 418-8666 (JP); SHIRATSUCHI Setsuko, Fujinomiya-shi Shizuoka 418-8666 (JP); HAMANO Mitsumasa, Fujinomiya-shi Shizuoka 418-8666 (JP)
(74) Representative: Hoffmann Eitle
(86) International application number: PCT/JP2015/062573
(87) International publication number: WO 2015/178166

(56) References cited:
- WO-A1-2013/026961
- WO-A1-2014/119626
- WO-A1-2015/147206
- JP-A- 2001 040 294
- JP-A- 2008 127 372
- JP-A- 2012 158 116
- US-A1- 2004 146 567
- US-A1- 2010 136 073
- US-A1- 2011 259 571

## Description

### BACKGROUND OF THE INVENTION

### 1. Field of the Invention

The present invention relates to an instrument, a protective sheet, and an antibacterial film. Specifically, the present invention relates to an instrument having a hydrophilic processed portion on at least a portion of an outer surface thereof, a protective sheet, and an antibacterial film in which at least a portion is hydrophilic.

### 2. Description of the Related Art

Contaminants adhere to the surface of medical devices such as radiographic imaging devices, particularly, a portable radiographic imaging device (electronic cassette), a Computed Radiography (CR) cassette, and a mammography device, that a plurality of patients or health professionals contact. If the medical devices are left as they are in such a state, bacteria will multiply. In order to inhibit the bacterial multiplication, the surface of the medical devices is appropriately sterilized using a disinfectant such as an aqueous ethanol solution or an aqueous sodium hypochlorite solution, but in some cases such as a case where the contaminants are fixed onto the surface, washing sterilization is not enough. Therefore, it is desired that the contaminants are easily wiped off.

For example, JP2012-132703A discloses a technique of mounting a buffer material, which has undergone waterproofing processing, on an outer surface of a medical instrument or device. This technique has an advantage of hindering the adherence of contaminants.

However, because a sterilization effect of a disinfectant solution is exerted only when the disinfectant solution stays for a certain period of time, there is a likelihood that a sterilization effect will not be sufficiently exerted on a surface that repels the disinfectant solution as in the technique disclosed in JP2012-132703A.

As means for solving the above problem, JP2010-503737A suggests a hydrophilic and antibacterial coating for devices inserted into the body.

JP2010-503737A discloses a coating obtained by curing a compound containing a hydrophilic polymer, a photo-initiator, metallic silver (Ag)-containing particles, and a carrier solution. The coating obtained from the compound disclosed in JP2012-503737A has both of lubricating properties and antibacterial activities. JP2010-503737A describes that the coating on an outer surface of a medical instrument such as a catheter is smooth in a moistened state. That is, JP2010-503737A describes that, when being wet, the coating is smooth such that the medical instrument can be inserted into a target portion of the body without causing a damage and causing a subject to experience an unacceptable level of pain. Furthermore, JP2010-503737A describes that, in order to be defined as being "wet", the coating needs to "contain water in an amount of equal to or greater than 10 wt% (with respect to a dry weight of the coating) so as to become smooth." Herein, wt% is % by mass and shows a proportion of water based on mass.

Meanwhile, in recent years, in view of ease of use, the number of medical devices having a touch panel has increased. Although these devices are operated by only health professionals, a biological monitor and the like used in an Intensive Care Unit (ICU) and the like are frequently touched by people, and hence contaminants easily adhere thereto. As a device that many unspecified people contact, a KIOSK terminal (installation type information terminal) having a touch panel such as a returning patient reception unit has become widespread in hospitals.

In addition, instruments having a touch panel are also being widespread in many electronic instruments such as mobile phones, mobile terminals, and navigation systems of automobiles.

These touch panels are required to be sanitary and to provide a long-term visibility which is original performance thereof. That is, the touch panels are required to provide visibility that does not easily deteriorate due to fingerprints, scratch, and the like.

As means for solving the above problems, JP2008-213206A suggests an antibacterial film forming solution for forming transparent article, in which an antibacterial film excellent in both of antibacterial properties and transparency is formed, and an antibacterial film.

JP2008-213206A discloses transparent article with an antibacterial film which contains silicon oxide as a main component on a glass board and fine silver particles and/or silver ions and may contain a hydrophilic polymer and in which a transparent silver-containing antibacterial film is formed. The transparent article with an antibacterial film disclosed in JP2008-213206A has excellent characteristics such as transparency, abrasion resistance, and antibacterial properties. Furthermore, JP2008-213206A describes that the antibacterial film forming solution disclosed in the document makes it possible to obtain transparent article with an antibacterial film having the above characteristics. In addition, JP2008-213206A describes that the transparent article with an antibacterial film disclosed in the document can be used in a display portion of an electronic instrument such as a mobile phone, a touch panel thereof, and the like.

In view of sterilization, the technique described in JP2012-132703A is poor in wettability of a disinfectant solution with respect to a surface of a medical device. That is, because the disinfectant solution is repelled, it is not easy for the disinfectant solution to stay for a long period of time on an outer surface of a medical device, and as a result, a sterilization effect is highly unlikely to be sufficiently exerted on bacteria.

Inversely, because the technique described in JP2010-503737A is for a medical instrument inserted into the body, a property of being able to contain moisture in an amount of equal to or greater than 10 wt% that is a property close to that of hydrogel is required such that smoothness of a moistened state is maintained. Therefore, the coating is inappropriate as a surface that is usually brought into contact with and operated by a human being.

In addition, the technique described in JP2008-213206A is basically for forming a silver-containing antibacterial inorganic film, which contains silicon oxide as a main component, on a transparent glass board by a sol-gel method. Although JP2008-213206A discloses that a hydrophilic polymer may be added, the technique is not applicable to a surface of a medical device because it is not for forming a silver-containing antibacterial organic film.

It is known that antibacterial processing is performed on a surface of devices, such as those located in a hospital environment or a public place, that unspecified many people contact. In a case where different people continuously contact the devices, if the antibacterial effect is weak, bacteria adhere to human beings contacting the devices after a carrier, and hence the intended effect of the antibacterial processing is unlikely to be obtained. If simply the antibacterial effect is sought for, a disinfectant or the like may be caused to present on the surface at a high concentration, but the strong disinfectant may cause harms such as rash and inflammation to human beings contacting the devices. Therefore, a strong antibacterial effect that is safe for a biological body is required.

The present invention has been made for solving the above problems, and an object thereof is to provide an instrument having excellent hydrophilicity and antibacterial properties, a protective sheet, and an antibacterial film.

In order to achieve the aforementioned object, an instrument as a first aspect of the present invention is an instrument comprising a hydrophilic processed portion on at least a portion of an outer surface thereof, in which the hydrophilic processed portion contains a hydrophilic polymer and a silver-containing antibacterial agent, a water contact angle of a surface of the hydrophilic processed portion is equal to or less than 80°, and an amount of silver ions per unit area of the hydrophilic processed portion of the hydrophilic portion that is measured by the following extraction test is preferably equal to or greater than 15 ng/cm².

Extraction condition: A 1/500 normal nutrient broth medium specified in JIS Z 2801:2010 is used as an extractant, and a temperature of the extractant is controlled within a range of 35 ± 1°C. The extractant is brought into contact with a surface of the hydrophilic processed portion (or the hydrophilic portion) for 1 hour, and an amount of silver ions extracted into the extractant is measured. The obtained value is divided by a contact area between the surface of the hydrophilic processed portion (or the hydrophilic portion) and the extractant, thereby obtaining an amount of silver ions per unit area. A unit of the amount of silver ions is ng, and a unit of the contact area is cm², and a unit of the amount of silver ions per unit area is ng/cm²

The instrument is preferably a touch panel, and the hydrophilic processed portion is preferably provided within the outer surface that a user contacts. At this time, it is possible to expect fingerprints and the like caused by the contact of fingers of human beings not to be easily noticed, and visibility not to be hindered.

In order to achieve the aforementioned object, a protective sheet as a second aspect of the present invention is a protective sheet comprising a hydrophilic processed portion on at least a portion of an outer surface thereof, in which the hydrophilic processed portion contains a hydrophilic polymer and a silver-containing antibacterial agent, a water contact angle of the surface of the hydrophilic processed portion is equal to or less than 80°, and an amount of silver ions per unit area of the hydrophilic processed portion of the hydrophilic portion that is measured by the following extraction test is preferably equal to or greater than 15 ng/cm².

Extraction condition: A 1/500 normal nutrient broth medium specified in JIS Z 2801:2010 is used as an extractant, and a temperature of the extractant is controlled within a range of 35 ± 1°C. The extractant is brought into contact with a surface of the hydrophilic processed portion (or the hydrophilic portion) for 1 hour, and an amount of silver ions extracted into the extractant is measured. The obtained value is divided by a contact area between the surface of the hydrophilic processed portion (or the hydrophilic portion) and the extractant, thereby obtaining an amount of silver ions per unit area. A unit of the amount of silver ions is ng, and a unit of the contact area is cm², and a unit of the amount of silver ions per unit area is ng/cm².

In order to achieve the aforementioned object, an antibacterial film as a third aspect of the present invention is an antibacterial film in which at least a portion is hydrophilic. The antibacterial film comprises a hydrophilic portion that exhibits hydrophilicity and contains a hydrophilic polymer and a silver-containing antibacterial agent, in which a water contact angle of a surface of the hydrophilic portion is equal to or less than 80°, and an amount of silver ions per unit area of the hydrophilic processed portion of the hydrophilic portion that is measured by the following extraction test is preferably equal to or greater than 15 ng/cm².

Extraction condition: A 1/500 normal nutrient broth medium specified in JIS Z 2801:2010 is used as an extractant, and a temperature of the extractant is controlled within a range of 35 ± 1°C. The extractant is brought into contact with a surface of the hydrophilic processed portion (or the hydrophilic portion) for 1 hour, and an amount of silver ions extracted into the extractant is measured. The obtained value is divided by a contact area between the surface of the hydrophilic processed portion (or the hydrophilic portion) and the extractant, thereby obtaining an amount of silver ions per unit area. A unit of the amount of silver ions is ng, and a unit of the contact area is cm², and a unit of the amount of silver ions per unit area is ng/cm²

In the first, second, and third aspects of the present invention, the water contact angle of the surface of the hydrophilic processed portion or the hydrophilic portion is preferably equal to or less than 60°, and a water absorption rate of the hydrophilic processed portion or the hydrophilic portion is preferably lower than 10 wt%.

In the first, second, and third aspects of the present invention, hydrophilic processing is performed on at least either a portion of a surface of an instrument, for example, a medical device which needs to be subjected to washing sterilization or a portion of a surface of a protective sheet which is mounted on or bonded to a surface of a touch panel or the like of a display portion of an electronic instrument or the like, or alternatively, at least a portion of an antibacterial film is made hydrophilic. In this way, a water contact angle is sufficiently reduced and becomes equal to or less than 80. Consequently, as effects resulting from hydrophilicity, when the surface is washed with wet cloth which contains a disinfectant solution such as an aqueous ethanol solution, a wiper which has been dipped into a disinfectant solution, or running water, the disinfectant solution or moisture goes in between a contaminant and the hydrophilic processed surface or the hydrophilic surface. Therefore, it is much easier to remove the contaminant from the hydrophilic processed surface or the hydrophilic surface than from a non-hydrophilic processed portion or a non-hydrophilic surface, and a risk that the contaminant will remain can be significantly reduced. Furthermore, when being sterilized with a disinfectant solution such as an aqueous ethanol solution or an aqueous sodium hypochlorite solution after being washed, the surface is sufficiently wet with the disinfectant solution because wettability of the surface is high. Therefore, the disinfectant solution acts for a long period of time on the bacteria remaining on the surface, and as a result, it is possible to expect sufficient sterilization effects to be assured.

In the first, second, and third aspects of the present invention, both of the hydrophilicity and antibacterial properties are established. Accordingly, it is possible to expect an antibacterial agent to act on bacteria or the like that survive in a trace amount even after washing sterilization and to expect the inhibition of bacterial multiplication.

In the first, second, and third aspects of the present invention, a hydrophilic binder is prepared from a liquid-type coating agent containing at least a polyfunctional acryl monomer, a cross-linking agent and a silver particle antibacterial agent, and a structure having a plurality of functional groups is established. As a result, it is possible to obtain a hydrophilic processed portion or a hydrophilic portion (coating), which has a hardness of equal to or greater than HB (3H, 2H, H, F, and HB) and preferably equal to or greater than F (3H, 2H, H, and F) in terms of pencil hardness, and to prepare a coating in which both of strong hydrophilicity and antibacterial properties are established. Owing to the effect resulting from the strong hydrophilicity of the prepared coating, the components constituting fingerprints wet and spread over the surface, and deterioration of visibility is effectively prevented. It is understood that, as a result, the coating of the present invention has excellent hard coat properties and fingerprint resistance.

That is, in the first, second, and third aspects described above, the hydrophilic processed portion or the hydrophilic portion is preferably formed of a coating agent containing at least a polymerizable compound having a hydrophilic group and two or more (meth)acryl groups, a cross-linking agent and an antibacterial agent, and the hydrophilic group is a polyoxyethylene group.

The antibacterial agent preferably contains at least silver-supporting ceramic particles or silver particles.

According to the present invention, hydrophilicity and antibacterial properties become excellent, washing and sterilization effects become strong, and a strong sterilization effect can be maintained for a long period of time. Furthermore, an antibacterial effect after washing and sterilization remains strong, and hence bacterial multiplication can be inhibited. In addition, fingerprints or the like caused by the contact of fingers or the like of human beings are not easily noticed, and hence visibility can be assured without deterioration.

### BRIEF DESCRIPTION OF THE DRAWINGS

Fig. 1 is a perspective sectional view showing a portion of a portable radiographic imaging device as an instrument according to an embodiment of the present invention.
Fig. 2 is a schematic longitudinal sectional view of the portable radiographic imaging device shown in Fig. 1.
Fig. 3 is a perspective view schematically showing the entirety of another portable radiographic imaging device as an instrument according to another embodiment of the present invention.
Fig. 4 is a perspective view schematically showing main portions of a mammography device as an instrument according to another embodiment of the present invention.
Fig. 5 is a perspective view schematically showing main portions of a radiographic imaging device for upright radiography as an instrument according to another embodiment of the present invention.
Fig. 6 is a sectional view schematically showing a display device using a touch panel as an instrument according to another embodiment of the present invention.

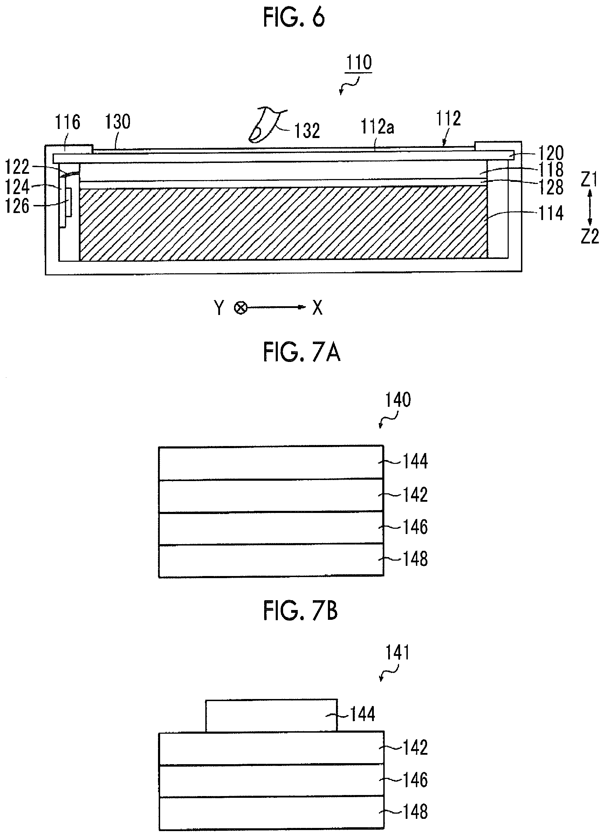

Each of Figs. 7A and 7B is a sectional view schematically showing a protective sheet according to another embodiment of the present invention.

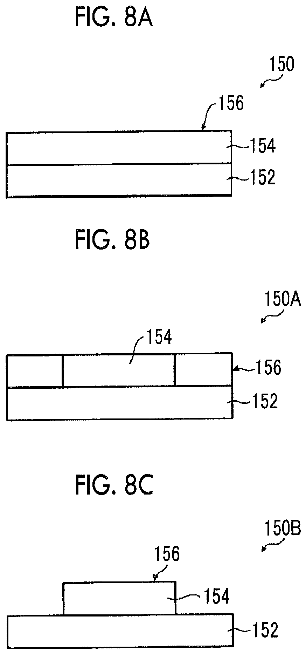

Each of Figs. 8A, 8B, and 8C is a sectional view schematically showing an antibacterial substrate with an antibacterial film according to another embodiment of the present invention.

### DESCRIPTION OF THE PREFERRED EMBODIMENTS

Hereinafter, an instrument, a protective sheet, and an antibacterial film according to the present invention will be specifically described by explaining preferred embodiments with reference to the attached drawings.

In the following section, as the instrument, a medical instrument will be described for example, but the present invention is not limited to the medical instrument and can be applied to instruments which are not for medical use.

In the present specification, a range of numerical values described using "to" means a range including numerical values listed before and after "to" as a lower limit and an upper limit respectively. Furthermore, because the drawings are made such that constituents are clearly illustrated, the sizes of the constituents in the drawings are different from the actual sizes thereof in some cases. However, it goes without saying that the drawings do not change the gist of the present invention.

### (First embodiment)

### (Portable radiographic imaging device)

First, a portable radiographic imaging device which is a medical instrument as a first embodiment according to the present invention will be described below.

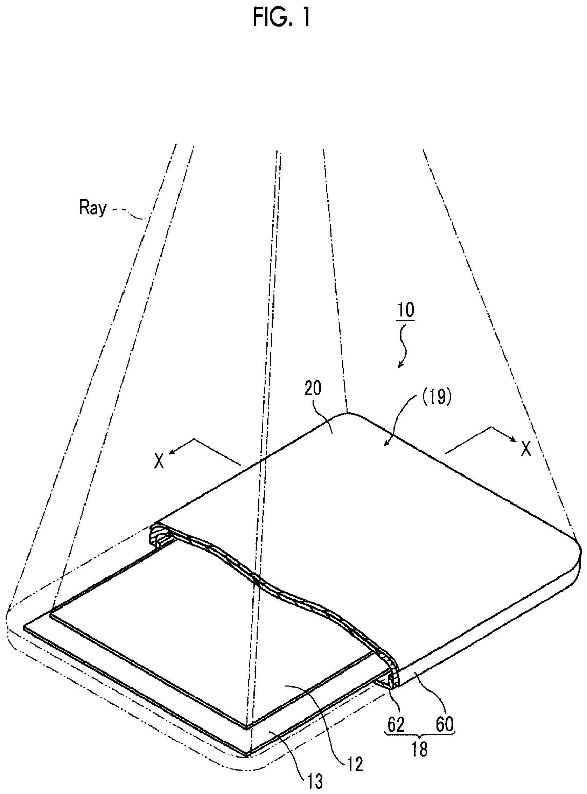

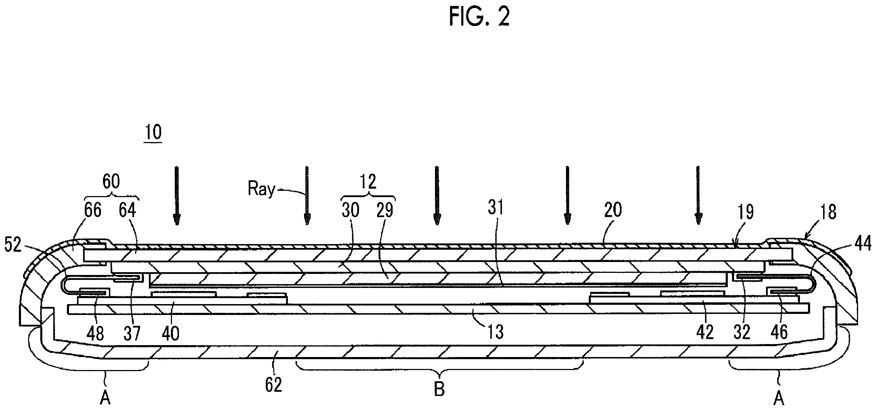

Fig. 1 is a perspective sectional view showing a portion of a portable radiographic imaging device (so-called electronic cassette) 10 according to the first embodiment. The portable radiographic imaging device 10 is a type of radiographic imaging device. Fig. 2 is a schematic longitudinal sectional view of the portable radiographic imaging device 10 and shows a section taken long the line X-X of Fig. 1. In the portable radiographic imaging device 10, an external surface of a housing 18 forms an outer surface in the present invention. In the inside of the housing 18, from an irradiation surface 19 side irradiated with radiation Ray, a radiation detector 12, which detects the radiation Ray having been transmitted through a patient not shown in the drawing, and a control board 13 which will be described later are provided in this order. Within the irradiation surface 19, a region in which a radiograph is imaged by the radiation detector 12 is an imaging region.

The radiation detector 12 is constituted with a scintillator 29 which is formed of Gadolinium Oxysulfide (GOS), Cesium Iodide (CsI), or the like and is bonded to a surface of a Thin Film Transistor (TFT) active matrix board (hereinafter, referred to as a TFT board) 30. In order to prevent the light generated by the scintillator 29 from leaking to the outside, the TFT board 30 may have a light screen element 31, which blocks the generated light, on a surface that is on the side opposite to the surface to which the scintillator 29 is bonded.

In the radiation detector 12, the radiation Ray such as an X-ray that is radiated from a radiation source not shown in the drawing is converted into light by the scintillator 29. The generated light enters sensor portions provided in the TFT board 30. The sensor portions receive the light generated from the scintillator 29 and accumulate a charge. Each of the sensor portions is provided with a TFT switch. When the TFT switch is turned ON, according to the amount of charges accumulated in the sensor portion, an electric signal (image signal) showing a radiograph flows in a signal line.

One end of the radiation detector 12 in a signal wiring direction is provided with a plurality of parallel connectors 32 for wire connection, and the other end of the radiation detector 12 in a scanning wiring direction is provided with a plurality of parallel connectors 37. The connectors 32 are connected to signal wiring, and the connectors 37 are connected to scanning wiring.

The control board 13 includes a scan signal control circuit 40 and a signal detection circuit 42. The scan signal control circuit 40 is provided with a connector 48, and the connector 48 is electrically connected to one end of a flexible cable 52. The other end of the flexible cable 52 is electrically connected to the connectors 37. Due to this constitution, the scan signal control circuit 40 can output control signals to each scanning wiring for turning the TFT switch ON/OFF.

The signal detection circuit 42 is provided with a plurality of connectors 46, and the connectors 46 are electrically connected to one end of a flexible cable 44. The other end of the flexible cable 44 is electrically connected to the connectors 32. The signal detection circuit 42 has a built-in amplification circuit, which amplifies the input electric signals, for each signal wiring. The charge accumulated in each sensor portions is input into each signal wiring, and the electric signal from each signal wiring is amplified by the amplification circuit through the signal detection circuit 42 and used as information on each pixel constituting an image.

The housing 18 has the built-in control board 13 which is in the form of a rectangular flat plate, performs various types of control such as the control of the imaging operation of the radiation detector 12 or the control of the communication with external devices, and is superimposed on the radiation detector 12 as shown in Fig. 2. In the present embodiment, the radiation detector 12 is disposed such that the TFT board 30 contacts an inner surface of the housing 18 on the irradiation surface 19 side.

In the housing 18, a front panel 60, which is disposed on a front surface side irradiated with the radiation Ray, in other words, on the side contacting a subject is provided to face a back panel 62 (back surface portion) which is disposed on a side opposite to the subject. The front panel 60 is constituted with a top panel 64 and a holding portion 66 that holds the top panel 64. A surface of the top panel 64 on the back panel 62 side is provided with the radiation detector 12. At both ends of Fig. 2 in a horizontal direction, the holding portion 66 is curved toward the back panel 62 side so as to form a portion of a lateral surface portion. Furthermore, at both ends of Fig. 2 in a horizontal direction, the back panel 62 curves toward the front panel 60 side so as to form a portion of the lateral surface portion. That is, the back surface portion of the housing 18 and a portion of the lateral surface portion are integrally formed. Herein, it is not necessary for the back surface portion and only a portion of the lateral surface portion to be integrally formed. The entirety of the lateral surface portion and the back surface portion may be integrally formed. At this time, the number of seams of the housing can be reduced, and thus wiping properties are improved.

In the present embodiment, the top panel 64 is formed of carbon, and accordingly, it is possible to assure strength while inhibiting the absorption of the radiation Ray. The holding portion 66 and the back panel 62 are formed of an ABS resin.

In the aforementioned constitution, a hydrophilic processed portion 20 is provided on an outer surface of the front panel 60. The outer surface provided with the hydrophilic processed portion 20 may include at least the irradiation surface 19 that a subject as a patient (not shown in the drawing) contacts at the time of imaging within the outer surface of the housing 18, that is, at least the outer surface of the top panel 64 and a portion of the holding portion 66 on the radiation source side.

Furthermore, for example, it is preferable to perform hydrophilic processing on a surface of the front panel 60 on the irradiation surface 19 side such that the hydrophilic processed portion 20 is provided on the irradiation surface 19. In addition, it is preferable to perform water repellent processing on an outer surface of the back panel 62 on the side opposite to the irradiation surface 19 (the side opposite to the subject), particularly, on the portion (portion indicated by a reference character A) of the outer surface on the outer circumferential side of the back panel 62, that is, a portion A of the outer surface on the outer circumferential side that starts from the seam between the back panel 62 and the holding portion 66 of the front panel 60 constituting a portion of the lateral surface portion of the housing 18 and reaches the region of the edge of the back panel 62 constituting a portion of the back surface portion of the housing 18, such that a water-repellent processed portion is provided.

In this way, if the water-repellent processed portion is provided on the outer surface on the outer circumference side (the portion A on both sides) of the back panel 62, a coefficient of friction of the outer surface on the outer circumference side is reduced, and hence the portable radiographic imaging device (electronic cassette) 10 can be easily inserted under the subject such as a patient.

In a case where corners of the electronic cassette incline (curve) as the holding portion 66 of the housing 18 of the portable radiographic imaging device (electronic cassette) 10 shown in Fig. 2, a contaminant may drip, and thus the periphery of the electronic cassette may be contaminated. In the present invention, the hydrophilic processed portion 20 is provided on the outer surface of the front panel 60 on the side of the irradiation surface 19. Accordingly, wettability of the surface of the front panel 60 is improved, a contaminant can be prevented from dripping from the holding portion 66 of the front panel 60, and the diffusion of the contaminant can be prevented.

In addition, embossing processing may be performed on a central portion B of the outer surface of the back panel 62 constituting the back surface portion of the housing 18. If an embossed structure is established in the central portion B, the electronic cassette 10 can be easily inserted under a subject such as a patient.

### (Hydrophilic processed portion)

The hydrophilic processed portion 20 contains at least a hydrophilic polymer and a silver-containing antibacterial agent.

Hereinafter, materials contained in the hydrophilic processed portion 20 will be specifically described.

### (Hydrophilic polymer)

The hydrophilic polymer is a polymer having a hydrophilic group.

The type of the hydrophilic group is not particularly limited, and examples thereof include a polyoxyalkylene group (for example, a polyoxyethylene group, a polyoxypropylene group, or a polyoxyalkylene group in which an oxyetylene group and an oxypropylene group are bonded to each other in the form of a block copolymer or a random copolymer), an amino group, a carboxyl group, an alkali metal salt of a carboxyl group, a hydroxy group, an alkoxy group, an amide group, a carbamoyl group, a sulfonamide group, a sulfamoyl group, a sulfonic acid group, an alkali metal salt of a sulfonic acid group, and the like. Among these, a polyoxyethylene group is preferable.

The structure of a main chain of the hydrophilic polymer is not particularly limited, and examples thereof include polyurethane, a poly(meth)acrylic acid ester, polystyrene, polyester, polyamide, polyimide, polyurea, and the like.

Conceptually, the poly(meth)acrylic acid ester includes both of a polyacrylic acid ester and a polymethacrylic acid ester.

One of the examples of preferred embodiments of the hydrophilic polymer includes a polymer obtained by polymerizing a monomer having the aforementioned hydrophilic group.

The monomer having a hydrophilic group is a polymerizable compound having the aforementioned hydrophilic group and a polymerizable group. The definition of the hydrophilic group is as described above.

The number of hydrophilic groups in the monomer having a hydrophilic group is not particularly limited. In view of making the hydrophilic processed portion exhibit stronger hydrophilicity, the number of the hydrophilic groups is preferably equal to or greater than 2, more preferably 2 to 6, and even more preferably 2 or 3.

The type of the polymerizable group is not particularly limited, and examples thereof include a radically polymerizable group, a cationically polymerizable group, an anionically polymerizable group, and the like. Examples of the radically polymerizable group include a (meth)acryl group, an acrylamide group, a vinyl group, a styryl group, an allyl group, and the like. Examples of the cationically polymerizable group include a vinyl ether group, an oxiranyl group, an oxetanyl group, and the like. Among these, a (meth)acryl group is preferable.

Conceptually, the (meth)acryl group includes both of an acryl group (acryloyl group) and a methacryl group (methacryloyl group).

The number of polymerizable groups in the monomer having a hydrophilic group is not particularly limited. In view of further improving mechanical strength and hard coat properties of the obtained hydrophilic processed portion, the number of polymerizable groups is preferably equal to or greater than 2, more preferably 2 to 6, and even more preferably 2 or 3.

That is, in the present invention, in view of hard coat properties, it is preferable that the monomer has two or more (meth)acryl groups as polymerizable groups.

One of the examples of preferred aspects of the monomer having a hydrophilic group includes a compound represented by the following Formula (1).

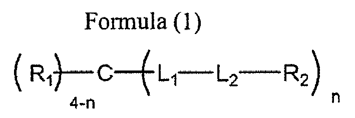

In Formula (1), R₁ represents a substituent. The type of the substituent is not particularly limited. Examples of the substituent include known substituents such as a hydrocarbon group (for example, an alkyl group or an aryl group), which may have a heteroatom, and the aforementioned hydrophilic group.

R₂ represents a polymerizable group. The definition of the polymerizable group is as described above.

L₁ represents a single bond or a divalent linking group. The type of the divalent linking group is not particularly limited, and examples thereof include -O-, -CO-, -NH-, -CO-NH-, -COO-, -O-COO-, an alkylene group, an arylene group, a heteroaryl group, and a combination of these.

L₂ represents a polyoxyalkylene group. The polyoxyalkylene group refers to a group represented by the following Formula (2).

Formula (2) *-(OR₃)ₘ-*

In Formula (2), R₃ represents an alkylene group (for example, an ethylene group or a propylene group). m represents an integer of equal to or greater than 2. m is preferably 2 to 10, and more preferably 2 to 6. * represents a binding position.

n represents an integer of 1 to 4.

In order to obtain a hydrophilic polymer, the aforementioned monomer having a hydrophilic group may be used in combination with other monomers, particularly, monomers including polyfunctional monomers. That is, it is preferable to use a hydrophilic polymer which is obtained by copolymerizing the monomer having a hydrophilic group and other monomers (monomers including polyfunctional monomers other than the monomer having a hydrophilic group).

The type of other monomers is not particularly limited as long as they include polyfunctional monomers, and known monomers can be appropriately used as long as they have a polymerizable group. The definition of the polymerizable group is as described above.

Among the monomers, a polyfunctional monomer having two or more polymerizable groups is preferably used, because then the mechanical strength of the hydrophilic processed portion, that is, hard coat properties are further improved. The polyfunctional monomer acts as a so-called cross-linking agent.

The number of polymerizable groups contained in the polyfunctional monomer is not particularly limited. In view of further improving mechanical strength and hard coat properties of the hydrophilic processed portion and in view of handleability, the number of polymerizable groups is preferably 2 to 10 and more preferably 2 to 6.

Examples of the polyfunctional monomer include trimethylolpropane triacrylate, tetramethylolmethane tetraacrylate, dipentaerythritol hexaacrylate, and pentaerythritol tetraacrylate.

A mixing ratio (mass of hydrophilic monomer/ mass of other monomers) of the hydrophilic monomer to other monomers (particularly, polyfunctional monomers) is not particularly limited. In view of making it easy to control hydrophilicity of the hydrophilic processed portion and in view of a necessity of assuring hard coat properties, the mixing ratio is preferably 0.01 to 15 and more preferably 0.1 to 15.

It is preferable that the hydrophilic processed portion contains the aforementioned hydrophilic polymer as a main component. Herein, the main component refers to a hydrophilic polymer of which the content is equal to or greater than 50 wt% with respect to a total mass of the hydrophilic processed portion. The content of the hydrophilic polymer is preferably equal to or greater than 70 wt%, and more preferably equal to or greater than 90 wt%.

### (Antibacterial agent)

The hydrophilic processed portion contains at least one kind of silver-containing antibacterial agent. The type of the antibacterial agent contained in the hydrophilic processed portion is not particularly limited as long as it is a silver-containing antibacterial agent, and known antibacterial agents can be used. As the antibacterial agent, those exerting a bactericidal effect on pathogenic bacteria represented by *Staphylococcus aureus* and *E. coli* are preferably used.

The silver-containing antibacterial agent (hereinafter, referred to as a silver-based antibacterial agent as well) should contain silver (silver particles), and the type thereof is not particularly limited. Furthermore, the form of the silver is not particularly limited, and for example, the silver is contained in the antibacterial agent in the form of metallic silver, a silver ion, a silver salt (including a silver complex), and the like. Preferred examples of the silver-based antibacterial agent include inorganic antibacterial agent containing silver particles, which slowly release silver ions, or silver, such as inorganic antibacterial agent obtained by causing silver or silver ions to be supported on a support. In the present specification, a silver complex is included in the scope of a silver salt.

Examples of the silver salt include silver acetate, silver acetylacetonate, silver azide, silver acetylide, silver arsenate, silver benzoate, silver hydrogen fluoride, silver bromate, silver bromide, silver carbonate, silver chloride, silver chlorate, silver chromate, silver citrate, silver cyanate, silver cyanide, silver (cis,cis-1,5-cyclooctadiene)-1,1,1,5,5,5-hexafluoroacetylactonate, silver diethyldithiocarbamate, silver (I) fluoride, silver (II) fluoride, silver 7,7-dimethyl-1,1,1,2,2,3,3-heptafluoro-4,6-octanedioate, silver hexafluoroantimonate, silver hexafluoroarsenate, silver hexafluorophosphate, silver iodate, silver iodide, silver isothiocyanate, potassium silver cyanide, silver lactate, silver molybdate, silver nitrate, silver nitrite, silver (I) oxide, silver (II) oxide, silver oxalate, silver perchlorate, silver perfluorobutyrate, silver perfluoropropionate, silver permanganate, silver perrhenate, silver phosphate, silver picrate monohydrate, silver propionate, silver selenate, silver selenide, silver selenite, silver sulfadiazine, silver sulfate, silver sulfide, silver sulfite, silver telluride, silver tetrafluoroborate, silver tetraiodomercurate, silver tetratungstate, silver thiocyanate, silver p-toluenesulfonate, silver trifluoromethanesulfonate, silver trifluoroacetate, silver vanadate, and the like.

An example of the silver complex include a histidine-silver complex, a methionine-silver complex, a cysteine-silver complex, an aspartic acid-silver complex, a pyrrolidone carboxylic acid-silver complex, an oxotetrahydrofuran carboxylic acid-silver complex, an imidazole-silver complex, or the like.

Examples of the silver-based antibacterial agent include an organic silver-based antibacterial agent such as the aforementioned silver salt (silver complex) and an inorganic silver-based antibacterial agent containing a support which will be described later. The type of the silver-based antibacterial agent is not particularly limited.

Among the silver-based antibacterial agents, in view of further improving light fastness of the hydrophilic processed portion and/or improving antibacterial properties (hereinafter, simply described as "in view of further improving effects of the present invention" as well), a silver-supporting support including a support and silver supported on the support is preferable.

The type of the support is not particularly limited, and examples thereof include a silicate-based support, a phosphate-based support, an oxide (for example, glass), potassium titanate, and an amino acid.

Examples of the support include a zeolite-based antibacterial agent support, a calcium silicate-based antibacterial agent support, a zirconium phosphate-based antibacterial agent support, a calcium phosphate-based antibacterial agent support, a zinc oxide-based antibacterial agent support, a soluble glass-based antibacterial agent support, a silica gel-based antibacterial agent support, an activated carbon-based antibacterial agent support, a titanium oxide-based antibacterial agent support, a titania-based antibacterial agent support, an organic metal-based antibacterial agent support, an ion exchanger ceramic-based antibacterial agent support, a layered phosphate-quaternary ammonium salt-based antibacterial agent support, an antibacterial stainless steel support, and the like, but the support is not limited to these.

Specific examples of the support include calcium zinc phosphate, calcium phosphate, zirconium phosphate, aluminum phosphate, calcium silicate, activated carbon, activated alumina, silica gel, zeolite, hydroxyapatite, zirconium phosphate, titanium phosphate, potassium titanate, hydrous bismuth oxide, hydrous zirconium oxide, hydrotalcite, and the like. Examples of zeolite include natural zeolite such as chabazite, mordenite, erionite, and clinoptilolite, and synthetic zeolite such as type A zeolite, type X zeolite, and type Y zeolite.

As the support, in view of further improving effects of the present invention, so-called ceramics are preferable.

An average particle size of the aforementioned silver-supporting support is not particularly limited. In view of further improving effects of the present invention, the average particle size of the silver-supporting support is preferably 0.1 to 10 µm, and more preferably 0.1 to 2 µm. The average particle size is a value obtained by measuring diameters of at least 10 random silver-supporting supports by using an microscope and calculating an arithmetic mean thereof.

A content of silver in the silver-based antibacterial agent is not particularly limited. For example, in a case of the aforementioned silver-supporting support, the content of silver is preferably 0.1 to 10 wt% and more preferably 0.3 to 5 wt% with respect to a total mass of the silver-supporting support.

Among the above antibacterial agents, silver particles or ceramic particles supporting silver (silver ceramic particles) are preferable because these have a strong antibacterial effect. More specifically, examples thereof include silver zeolite in which silver is supported on zeolite as a silicate-based support and an antibacterial agent in which silver is supported on silica gel.

Examples of particularly preferred commercially available silver zeolite-based antibacterial agents include "ZEOMIC" from Sinanen Zeomic Co., Ltd., "SILWELL" from FUJI SILYSIA CHEMICAL LTD., "BACTENON" from JAPAN ELECTRONIC MATERIALS CORPORATION, and the like. In addition, "NOVARON" from TOAGOSEI CO., LTD. in which silver is supported on inorganic ion exchanger ceramics, "ATOMY BALL" from Shokubai Kasei Kogyo Co., and "SAN-AI BACK P" as a triazine-based antibacterial agent are also preferable. As silver particles, "NANOSILVER" from Japan Ion Corporation. can be selected. Furthermore, it is also possible to select "BACTEKILLER" or "BACTELITE" from Fuji Chemical Industries, Ltd. composed of silver ceramic particles obtained by chemically bonding silver to ceramics.

In the present invention, in addition to the silver-containing antibacterial agent, other known antibacterial agents may be used in combination. Examples of other known antibacterial agents include an inorganic antibacterial agent not containing silver or an organic antibacterial agent (preferably a water-soluble organic antibacterial agent).

Examples of the organic antibacterial agent include a phenol ether derivative, an imidazole derivative, a sulfone derivative, a N-haloalkylthio compound, an anilide derivative, a pyrrole derivative, a quaternary ammonium salt, a pyridine-based compound, a triazine-based compound, a benzisothiazoline-based compound, an isothiazoline-based compound, and the like.

More specifically, examples of the organic antibacterial agent include 1,2-benzisothiazolin-3-one, N-fluorodichloromethylthio-phthalimide, 2,3,5,6-tetrachloroisophthalonitrile, N-trichloromethylthio-4-cyclohexene-1,2-dicarboximide, copper 8-quinolinate, bis(tributyltin)oxide, 2-(4-thiazolyl)benzimidazole <hereinafter, described as TBZ>, methyl 2-benzimidazole carbamate hereinafter, described as BCM>, 10,10'-oxybisphenoxarsine <hereinafter, described as OBPA>, 2,3,5,6-tetrachloro-4-(methylsulfonyl)pyridine, zinc bis(2-pyridylthio-1-oxide) <hereinafter, described as ZPT>, N,N-dimethyl-N'-(fluorodichloromethylthio)-N'-phenylsulfonamide<dichlofluanide>, poly-(hexamethylenebiguanide)hydrochloride, dithio-2,2'-bis(benzmethylamide), 2-methyl-4,5-trimethylene-4-isothiazolin-3-one, 2-bromo-2-nitro-1,3-propanediol, hexahydro-1,3-tris-(2-hydroxyethyl)-S-triazine, p-chloro-m-xylenol, and the like, but the organic antibacterial agent is not limited to these.

These organic antibacterial agents can be appropriately selected and used in consideration of hydrophilicity, water resistance, sublimation properties, safety, and the like. Among these organic antibacterial agents, in view of hydrophilicity, antibacterial effects, and costs, 2-bromo-2-nitro-1,3-propanediol, TBZ, BCM, OBPA, or ZPT is preferable.

The organic antibacterial agent also includes a natural antibacterial agent. The natural antibacterial agent includes chitosan which is basic polysaccharide obtained by hydrolyzing chitin contained in crustacean such as crab or shrimp.

Examples of the inorganic antibacterial agent include mercury, copper, zinc, iron, lead, bismuth, and the like listed in descending order of the strength of the antibacterial effect. Examples of the inorganic antibacterial agent also include those obtained by causing a metal such as copper, zinc, or nickel or metal ions to be supported on a support. As the support, those described above can be used.

Among the above antibacterial agents, metal particles (particularly, copper particles are preferable) or organic antibacterial agents are preferable because these have a strong antibacterial effect. As the organic antibacterial agent, 2-bromo-2-nitro-1,3-propanediol, TPN, TBZ, BCM, OBPA, or ZPT is preferable.

As the most preferred aspect of the inorganic antibacterial agent used in combination with the silver-containing inorganic antibacterial agent, copper particles, which slowly release copper ions, and copper ceramic particles are preferable.

A content of the antibacterial agent in the hydrophilic processed portion is not particularly limited. In view of the balance between the removability of contaminants and antibacterial properties, the content of the antibacterial agent is preferably 0.001 to 15 wt%, more preferably 0.001 to 10 wt%, and even more preferably 0.001 to 5 wt%, with respect to a total mass of the hydrophilic processed portion.

In a case where other antibacterial agents are used as an antibacterial agent in addition to the silver-containing antibacterial agent, a total content of the antibacterial agents should satisfy the above range, and a content of other antibacterial agents may be equal to or less than 50 wt% and preferably equal to or less than 20 wt% with respect to a total amount of the antibacterial agents (or a silver-containing inorganic antibacterial agent).

In a case where silver particles are used as an antibacterial agent, a content of the antibacterial agent in the hydrophilic processed portion is preferably 0.001 to 5 wt%, more preferably 0.001 to 2 wt%, even more preferably 0.001 to 1 wt%, and particularly preferably 0.001 to 0.1 wt%, with respect to a total mass of the hydrophilic processed portion. If the content is equal to or greater than 0.001 wt%, the antibacterial effect can be further improved. If the content is equal to or less than 5 wt%, hydrophilicity is not reduced, temporal properties do not deteriorate, and antifouling properties are not negatively affected.

An average particle size of the silver particles is preferably 1 nm to 100 nm, and more preferably 1 nm to 20 nm. The smaller the particle size of the silver particles, the greater the ratio of surface area/volume, and as a result, antibacterial properties can be obtained with a smaller amount of silver particles.

The average particle size means a particle size at 50% in a cumulative particle size distribution measured by a laser diffraction·scattering method.

A content of the aforementioned silver-based antibacterial agent in the hydrophilic processed portion should satisfy the above range. In view of further improving effects of the present invention, it is preferable to incorporate the silver-containing antibacterial agent into the hydrophilic processed portion such that a content of silver with respect to a total mass of the hydrophilic processed portion becomes 0.001 to 20 wt% (more preferably 0.001 to 10 wt% and even more preferably 0.001 to 5 wt%).

Furthermore, in a case where an organic silver-based antibacterial agent is used as a silver-based antibacterial agent, a content of the antibacterial agent should also satisfy the above range. In view of further improving mechanical strength of the hydrophilic processed portion and further improving effects of the present invention, the content of the antibacterial agent is more preferably 1 to 5 wt% with respect to a total mass of the hydrophilic processed portion.

In a case where an inorganic silver-based antibacterial agent used as the silver-based antibacterial agent, a content of the antibacterial agent should also satisfy the above range. In view of further improving mechanical strength of the hydrophilic processed portion and further improving effects of the present invention, the content of the antibacterial is preferably 0.001 to 10 wt% and more preferably 0.01 to 5 wt% with respect to a total mass of the hydrophilic processed portion.

In a case where silver ceramic particles are used, if a content thereof is equal to or greater than 0.1 wt% with respect to a total mass of the hydrophilic processed portion, the antibacterial effect can be further improved. If the content is equal to or less than 10 wt%, hydrophilicity is not reduced, temporal properties do not deteriorate, and antifouling properties are not negatively affected.

An average particle size of the silver ceramic particles is preferably 0.1 µm to 10 µm, and more preferably 0.1 µm to 2 µm.

In a case where an organic antibacterial agent is used as an antibacterial agent in addition to the silver-containing antibacterial agent, in view of the balance between removability of contaminants and antibacterial properties, a content of the organic antibacterial agent with respect to a total mass of the hydrophilic processed portion is preferably 0.0005 to 2.5 wt%.

In the present invention, the antibacterial agent may not be exposed on a surface of the hydrophilic processed portion.

Furthermore, the hydrophilic processed portion may contain other components in addition to the aforementioned hydrophilic polymer and antibacterial agent.

In the present invention, the hydrophilic processed portion may contain a metal oxide-containing photocatalytic material in addition to the silver-containing antibacterial agent. The photocatalytic material also has an antibacterial activity as the aforementioned silver-containing antibacterial agent.

The type of the metal oxide contained in the photocatalytic material is not particularly limited, and examples thereof include TiO₂, ZnO, SrTiO₃, CdS, GaP, InP, GaAs, BaTiO₃, BaTiO₄, BaTi₄O₉, K₂NbO₃, Nb₂O₅, Fe₂O₃, Ta₂O₅, K₃Ta₃Si₂O₃, WO₃, SnO₂, Bi₂O₃, BiVO₄, NiO, Cu₂O, SiC, MoS₂, InPb, RuO₂, CeO₂, Ta₃N₅, and a layered oxide having at least one kind of element selected from Ti, Nb, Ta, and V. Among these, in view of further improving effects of the present invention, metal oxides having at least one kind of metal atom selected from the group consisting of Zn, Ti, Ni, W, Cu, Sn, Fe, Sr, and Bi are preferable.

In addition, in view of further improving effects of the present invention, TiO₂ or WO₃ is preferred as a metal oxide contained in the photocatalytic material.

An average particle size of the photocatalytic material is not particularly limited, and is preferably 1 nm to 2 µm, more preferably 10 nm to 1.5 µm, and even more preferably 20 nm to 1 µm. The average particle size is a value obtained by measuring diameters of at least 10 random photocatalytic materials by using a microscope and calculating an arithmetic mean thereof. In a case where the photocatalytic material is not a perfect circle, a major axis thereof is taken as the diameter.

Provided that an average thickness of the hydrophilic processed portion is denoted by T, and an average particle size of the photocatalytic material contained in the hydrophilic processed portion is denoted by Da, a ratio of the average thickness T to the average particle size Da (T/Da) is preferably T/Da ≤ 10,000, more preferably T/Da ≤ 1,000, and even more preferably T/Da ≤ 300. In a case where the hydrophilic processed portion contains two or more kinds of photocatalytic material, an average particle size of a photocatalytic material having the smallest average particle size is taken as Da.

A mass ratio of a mass of the silver-containing antibacterial agent to a mass of the photocatalytic material (mass of silver-containing antibacterial agent/mass of photocatalytic material) is preferably 0.01 to 20, more preferably 0.1 to 10, and even more preferably 0.3 to 3.

### (Water contact angle)

In order to improve the antibacterial effect, a water contact angle of a surface of the hydrophilic processed portion needs to be equal to or less than 80°. In view of further improving removability of contaminants, the water contact angle is preferably equal to or less than 60°, more preferably equal to or less than 30°, and even more preferably equal to or less than 15°.

The fact that the hydrophilicity of a binder contributes to slow releasing properties of silver ions is considered to be one of the factors improving the effects such as hydrophilicity and antibacterial properties and the antibacterial effect. In addition, presumably, such improvement may also result from an additional effect that due to the improvement of hydrophilicity of the surface, silver ions are easily ingested by bacteria due to the interaction or the like between the bacteria and the surface.

A lower limit of the water contact angle is not particularly limited. In view of the characteristics of the material used, the lower limit of the water contact angle is equal to or greater than 5° in many cases.

In the present specification, the water contact angle is measured based on a sessile drop method of JIS R 3257:1999. For measuring the water contact angle, LSE-ME1 (software 2win mini) manufactured by NiCK Corporation is used. More specifically, at room temperature (20°C), 2 µl of droplets of pure water are dropped onto a surface of the hydrophilic processed portion which is kept horizontal, and a contact angle at a point in time when 20 seconds has elapsed from the dropping is measured.

### (Amount of silver ions)

In the aforementioned electronic cassette 10 which is a medical instrument, the hydrophilic processed portion 20 is provided on an outer surface of the housing 18 thereof, for example, on an outer surface of the top panel 64 and the holding portion 66 of the front panel 60. When the top panel 64 or the holding portion 66 is used as a substrate onto which the hydrophilic processed portion 20 is to be provided, in the substrate with the hydrophilic processed portion that includes a substrate and the hydrophilic processed portion 20, an amount of silver ions per unit area that is measured by an extraction test which will be described later is preferably equal to or greater than 15 ng/cm², and desirably equal to or less than 100 ng/cm². In view of further improving effects of the present invention, the amount of silver ions is more preferably 15 to 75 ng/cm², and even more preferably 15 to 50 ng/cm².

In a case where the amount of silver ions is less than 15 ng/cm², antibacterial properties become poor in some cases, and in a case where the amount of silver ions is greater than 100 ng/cm², light fastness becomes poor in some cases. Accordingly, a preferred range of the amount of silver ions is limited as above.

Hereinafter, a method of an extraction test will be specifically described.

In the extraction test, a 1/500 normal nutrient broth medium specified in JIS Z 2801:2010 is used as an extractant, and a temperature of the extractant is controlled within a range of 35 ± 1°C. The extractant (amount: 9 mL) is brought into contact for 1 hour with a surface of a hydrophilic processed portion (area of hydrophilic processed portion: 4 cm² (2 cm × 2 cm)) in a substrate with a hydrophilic processed portion. As a method for bringing the hydrophilic processed portion into contact with the extractant, a method of dipping the substrate with a hydrophilic processed portion in the extractant is performed.

Then, after 1 hour, the substrate with a hydrophilic processed portion is recovered from the extractant, and an amount of silver ions (ng) extracted into the extractant is measured. The amount of silver ions in the extractant is measured using atomic absorption spectrometry (contrAA 700 manufactured by Analytik Jena AG) and determined from a calibration curve that is plotted in advance.

At the time of measuring the amount of silver ions, if necessary, it is preferable to add nitric acid (about 1 mL) to the extractant so as to improve stability of measurement.

Thereafter, by dividing the obtained amount of silver ions by a contact area (4 cm²) between the hydrophilic processed portion and the extractant, an amount of silver ions per unit area (ng/cm²) is calculated. The contact area between the hydrophilic processed portion and the extractant means an area in which the hydrophilic processed portion and the extractant contact each other when the hydrophilic processed portion is brought into contact with the extractant. For example, in Fig. 2, the contact area means an area of a main surface that is on the side opposite to the top panel 64 side or the holding portion 66 side that is a substrate of the hydrophilic processed portion 20.

The amount of silver ions obtained as above represents a degree of elution (extraction) of silver ions from the hydrophilic processed portion.

### (Vickers hardness)

In the evaluation of hard coat properties, A Vickers hardness (HV) is effective not as an index of scratch that can be evaluated by pencil hardness but as an index of vulnerability confirmed when a sharp object is pressed on a surface.

A Vickers hardness of the hydrophilic processed portion pressed under the following conditions is preferably equal to or greater than 1.33 and more preferably equal to or greater than 1.35. The Vickers hardness is desirably equal to or greater than 5 and more desirably equal to or greater than 10. An upper limit of the Vickers hardness is about 90.

Measurement conditions: by using a Vickers hardness tester (manufactured by Fischer Instruments K.K.), an average of n 10 which is a value at the time of measurement under a load applied at 20 mN/20 s for a loading time of 5 s is calculated.

### (Average thickness of hydrophilic processed portion)

An average thickness of the hydrophilic processed portion is not particularly limited. In view of removability of contaminants and antibacterial properties, the average thickness is preferably 0.5 µm to 20 µm, and more preferably 1 µm to 10 µm.

The average thickness of the hydrophilic processed portion is measured by the following method, for example. A sample piece including the hydrophilic processed portion is embedded in a resin, a section thereof is cut with a microtome, and the cut section is observed with a scanning electron microscope so as to measure the average thickness. The thicknesses in 10 random points in the hydrophilic processed portion are measured, and an arithmetic mean thereof is calculated.

The surface of the hydrophilic processed portion does not need to be subjected to a special surface treatment, and may be a flat surface prepared by a preparation method which will be described later.

### (Water absorption rate)

A water absorption rate of the hydrophilic processed portion is preferably less than 10 wt%, and more preferably equal to or less than 2 wt%.

In a case where the water absorption rate is equal to or greater than 10 wt%, an amount of moisture in the hydrophilic processed portion is too large, and hence the surface of the hydrophilic processed portion is in a state close to hydrogel. Consequently, the surface is inappropriate as a surface which is brought into contact with and operated by human beings. If a large amount of disinfectant solution remains as moisture in the hydrophilic processed portion, the disinfectant solution may cause harms such as rash and inflammation to human being contacting it.

In the present specification, a water absorption rate of the hydrophilic processed portion is a value with respect to a dry weight of the hydrophilic processed portion. The water absorption rate of the hydrophilic processed portion is measured as below.

One hundred samples with a hydrophilic processed portion (hereinafter, described as hydrophilic processed samples) obtained by forming a hydrophilic processed portion in a film having a predetermined dimension (10 cm × 10 cm) and 100 samples without a hydrophilic processed portion (hereinafter, described as non-hydrophilic processed samples) are prepared and used.

First, a weight of the hydrophilic processed portion is measured, and a weight difference between a hydrophilic processed sample and a non-hydrophilic processed sample is measured. At this time, by using the two kinds of sample, 100 each, an average thereof is determined.

Next, the hydrophilic processed sample is dipped into water with a temperature of 23°C for 24 hours, water drops other than moisture contained in the film are wiped off, and a weight of the hydrophilic processed sample is measured. Then, the sample is dried by being put into an oven with a temperature of 80°C for 24 hours, and then a weight thereof is measured. A weight difference of the hydrophilic processed sample before and after drying is an amount of water absorbed. A ratio (percentage) of the amount of water absorbed to the weight of the hydrophilic processed portion determined as above is taken as a water absorption rate of the hydrophilic processed portion.

In a case where an amount of water absorbed into the film without a hydrophilic processed portion is not negligible, an amount of water absorbed may be corrected by measuring the amount of water absorbed into the film. The non-hydrophilic processed sample is dipped into water with a temperature of 23°C for 24 hours. Water drops other than moisture contained in the film are wiped off, and a weight of the non-hydrophilic processed sample is measured. Then, the sample is dried by being put into an oven with a temperature of 80°C for 24 hours, and then a weight thereof is measured. A weight difference before and after drying is an amount of water absorbed into the film. Furthermore, a difference obtained by subtracting the amount of water absorbed into the film from the amount of water absorbed, which is a weight difference of the hydrophilic processed sample before and after drying, is a corrected amount of water absorbed into the hydrophilic processed portion. A ratio (percentage) of the corrected amount of water absorbed to the weight of the hydrophilic processed portion is a corrected water absorption rate of the hydrophilic processed portion.

### (Preparation of hydrophilic processed portion)

A method for preparing the aforementioned hydrophilic processed portion is not particularly limited, and known methods can be adopted. Examples of the method include a method of forming a hydrophilic processed portion through coating by using a composition containing the aforementioned hydrophilic polymer and antibacterial agent, a method of bonding a separately prepared polymer film containing a hydrophilic polymer and an antibacterial agent to a predetermined position, and the like.

Among these methods, in view of making it easier to adjust the thickness of the hydrophilic processed portion, the aforementioned method (coating method) is preferable in which a predetermined position is coated with a composition for forming a hydrophilic processed portion (hereinafter, simply referred to as a "composition" as well) containing a monomer having a hydrophilic group and an antibacterial agent so as to form a coating film, and the coating film is subjected to a curing treatment.

The composition contains the aforementioned monomer having a hydrophilic group and antibacterial agent. Furthermore, the composition may contain other components (other monomers described above and a solvent (water or an organic solvent)).

The composition may also contain a polymerization initiator. If the composition contains a polymerization initiator, polymerization more efficiently proceeds in the coating film, and thus a hydrophilic processed portion having excellent mechanical strength is formed. The type of the polymerization initiator is not particularly limited, and an optimal type is selected according to the method of the curing treatment. For example, a thermal polymerization initiator or a photopolymerization initiator is selected. More specifically, examples of the polymerization initiator include aromatic ketones such as benzophenone and phenylphosphine oxide, α-hydroxyalkylphenone-based compounds (BASF IRGACURE 184, 127, 2959, DAROCUR 1173, and the like), phenylphosphine oxide-based compounds (MAPO: BASF LUCIRIN TPO and BAPO: BASF IRGACURE 819), and the like.

A content of the polymerization initiator contained in the composition is not particularly limited. The content is preferably 0.1 to 15 wt%, and more preferably 1 to 6 wt%, with respect to a total mass of the monomer having a hydrophilic group and other monomers.

The coating method of the composition is not particularly limited, and known methods can be adopted.

Furthermore, the method of the curing treatment is not particularly limited, and examples thereof include a heating treatment or a light irradiation treatment.

The portable radiographic imaging device 10 according to the present embodiment is basically constituted as above. Next, the operation and effects thereof will be described.

In order to obtain a radiograph of a subject, first, at least the outer surface 20 of the portable radiographic imaging device 10 on the irradiation surface 19 side, preferably, the entirety of the outer surface of the housing 18 is cleaned. That is, the housing 18 is wiped with a wiper containing a disinfectant solution. As the disinfectant solution, an aqueous ethanol solution or an aqueous sodium hypochlorite solution is preferably used.

As described above, the outer surface of the housing 18 of the portable radiographic imaging device 10 is provided with a hydrophilic processed portion. Furthermore, in the hydrophilic processed portion, a water contact angle is equal to or less than 80° even in a dark place not irradiated with light. Therefore, even if the portable radiographic imaging device 10 is stored in a dark place, the outer surface of the housing 18 exhibits sufficient hydrophilicity.

Due to the hydrophilicity, the outer surface of the housing 18 is sufficiently wetted with the disinfectant solution. In other words, the disinfectant solution sufficiently wets and spreads over the outer surface of the housing 18. Accordingly, even if bacteria remain on the outer surface of the housing 18 at this point in time, the disinfectant solution contacts the bacteria for a long period of time. Furthermore, because the hydrophilic processed portion on the outer surface of the housing 18 contains an antibacterial agent, the antibacterial agent acts on the bacteria. Consequently, a bactericidal ability can be further improved compared to the related art, and bacterial multiplication can be inhibited.

That is, in a case where the portable radiographic imaging device 10 is stored in a dark place, the device can also be sterilized immediately after being taken out of the dark place.

In a state where the irradiation surface 19 of the housing 18 of the portable radiographic imaging device 10 cleaned as above is contacting a subject, a physician or a radiological technician (radiographer) irradiates an imaging site of the subject with the radiation Ray from a radiation source. The radiation Ray is transmitted through the imaging site of the subject, passes through the irradiation surface 19 of the portable radiographic imaging device 10, and reaches the scintillator 29 of the radiation detector 12.

The scintillator 29 emits fluorescence (visible light) in an amount according to the transmission amount of the radiation Ray. Meanwhile, in the sensor portions provided in the TFT board 30, a charge in an amount according to the amount (emission amount) of the fluorescence is generated and accumulated. The information on the charge is read out by the control portion, and as a result, a radiograph of the imaging site of the subject is obtained.

The portable radiographic imaging device 10 is used in an operating room, an emergency room, and the like in some cases. In these cases, the blood or body fluid of a patient (subject) is likely to adhere to the housing 18. In order to remove this type of contaminant, a method of washing the housing 18 with running water is considered. However, if doing so, a battery mounting portion or a connector connecting portion may be wetted with water, and thus the device may break down.

Therefore, after imaging ends, the housing 18 is wiped with a wiper containing a disinfectant solution such as an aqueous ethanol solution or an aqueous sodium hypochlorite solution. In this case, because the outer surface of the housing 18 is also provided with the hydrophilic processed portion 20 as described above, the disinfectant solution wets and spreads on the outer surface of the housing 18, and the outer surface is sufficiently wetted with the disinfectant solution.

Accordingly, in a case where a contaminant has adhered to the housing 18, water or the disinfectant solution goes in between the hydrophilic processed portion and the contaminant. As a result, the contaminant is easily detached from the housing 18. That is, the contaminant can be easily removed.

That is, the outer surface of the housing 18 is subjected to hydrophilic processing, and a water contact angle of the hydrophilic processed portion is sufficiently reduced. Therefore, when the surface is washed with wet cloth containing a disinfectant solution, a wiper dipped into a disinfectant solution such as an aqueous ethanol solution, or running water, moisture goes in between the contaminant and the surface of the hydrophilic processed portion. As a result, the contaminant is much more easily removed from the hydrophilic processed surface than from a non-hydrophilic processed surface, and a risk that the contaminant will remain can be significantly reduced.

Furthermore, owing to its high wettability, the surface is sufficiently wetted with the disinfectant solution, and the disinfectant solution stays on the outer surface of the housing 18 for a long period of time. Therefore, even if bacteria from the contaminant remain on the outer surface, the disinfectant solution contacts the bacteria for a long period of time, and hence a sufficient sterilization effect can be assured. In addition, because the hydrophilic processed portion on the outer surface of the housing 18 contains an antibacterial agent, both of hydrophilicity and antibacterial properties can be established, and the antibacterial agent acts on the bacteria and the like that survive in a trace amount even after washing sterilization. Accordingly, a bactericidal ability can be further improved compared to the related art, and bacterial multiplication can be inhibited.

As described above, if the outer surface of the housing 18 is provided with the hydrophilic processed portion, it is possible to remove the contaminant from the portable radiographic imaging device, which has a difficulty in being cleaned with running water in the related art, and to easily perform sterilization with an improved bactericidal ability in a case where sterilization is performed using a disinfectant solution.

According to the present embodiment, the housing 18 of the portable radiographic imaging device 10 which has been used for imaging can be easily cleaned. Furthermore, because the hydrophilic processed portion on the outer surface of the housing 18 contains an antibacterial agent, it is possible to obtain advantages that bacterial multiplication is inhibited, and the housing 18 remains clean for a long period of time.

### (Second embodiment)

### (Portable radiographic imaging device)

Next, a portable radiographic imaging device which is an instrument as a second embodiment according to the present invention will be described below. Unlike the first embodiment, the present embodiment has a so-called monocoque-type housing.

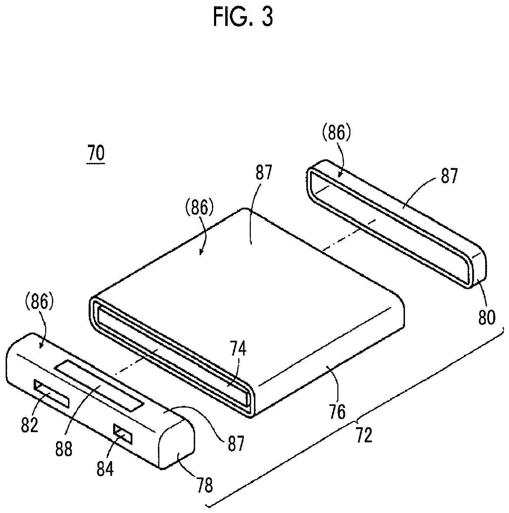

Fig. 3 is a perspective view schematically showing the entirety of a portable radiographic imaging device 70 as an instrument according to the second embodiment. In the portable radiographic imaging device 70, an external surface of a housing 72 forms an outer surface of the device, and a radiation detector 74 is accommodated in the housing 72.

The radiation detector 74 includes a scintillator, sensor portions, and the like not shown in the drawing. Furthermore, the radiation detector 74 is provided with a charge amplifier IC, a communication portion, and the like (none of these are shown in the drawing).

The housing 72 includes a body member 76 of which both ends in a longitudinal direction are open ends that are opened and a first cap member 78 and a second cap member 80 which close the open ends. The body member 76 has a cavity in the inside thereof and is in the form of a so-called cylinder. All of the body member 76, the first cap member 78, and the second cap member 80 may be constituted with a resin material that can transmit radiation.

The first cap member 78 is provided with a battery mounting portion 82 and an external instrument connecting portion such as a connector connecting portion 84. From a battery (not shown in the drawing) mounted on the battery mounting portion 82, a driving current is supplied, and through a connector (not shown in the drawing) mounted on the connector connecting portion 84, wire communication is performed between the portable radiographic imaging device 70 and external instruments. It goes without saying that wireless communication may be performed instead of the wired communication.

As shown in Fig 3, the first cap member 78 may be provided with a display portion 88 or the like. The display portion 88 is constituted with an LED lamp or the like, and is used for displaying the driving state and the like of an electronic cassette 70.

A surface on one end (surface on the radiation source side) of the body member 76, the first cap member 78, and the second cap member 80 constituting the housing 72 is an irradiation surface 86 irradiated with radiation which contacts a subject (not shown in the drawing) as a patient. Similarly to the aforementioned portable radiographic imaging device 10, at least the portion of the body member 76, the first cap member 78, and the second cap member 80 that contacts the subject (that is, the surface irradiated with radiation) is provided with a hydrophilic processed portion 87 composed of a hydrophilic layer. Herein, the hydrophilic processed portion may be provided not only in the portion of the body member 76, the first cap member 78, and the second cap member 80 that contacts the subject but also in the entirety of the outer surface that a radiographer can contact.

In this case, because the open ends of the body member 76 accommodating the radiation detector 74 in the inside thereof are closed by the first cap member 78 and the second cap member 80, a so-called monocoque-type housing 72 is formed, and the portable radiographic imaging device 70 is constituted. The body member 76 should be able to accommodate the radiation detector 74 in the inside thereof, and both ends thereof are not necessarily open ends. For example, only one end thereof may be an open end, and the open end may be closed by a cap member.

In the portable radiographic imaging device 70, the same effects as in the portable radiographic imaging device 10 according to the first embodiment are obtained. Herein, in a case where a portion contacting a subject is very far away from an external instrument connecting portion such as the connector connecting portion 84, for example, in a case where the portable radiographic imaging device 70 is a radiographic imaging device in which the external instrument connecting portion will not be wetted with water even when the portion contacting a subject is washed with water, the portion contacting a subject may be washed with running water. In this case, due to the presence of the hydrophilic processed portion, water also goes in between a contaminant and the outer surface. Accordingly, it is possible to easily remove the contaminant and to inhibit bacterial multiplication.

In the portable radiographic imaging device 70 shown in Fig. 3, the second cap member 80 can be mounted on the body member 76 through fitting, adhesion, or welding. In a case where a structure is adopted in which the first cap member 78 is detachably mounted through adhesion or welding, the portion other than the first cap member 78 can be dipped into a washing solution. At this time, it is preferable to cap the battery mounting portion 82 and the connector connecting portion 84 with an elastomer not shown in the drawing so as to prevent these portions from being accidentally wetted with. Furthermore, it is preferable to make the first cap member 78 waterproof by using an O-ring or the like not shown in the drawing in advance.

In this case, because the housing 72 is also provided with the hydrophilic processed portion, it is possible to easily remove the contaminant and to inhibit bacterial multiplication.

In the present embodiment, the first cap member 78 may be provided with a detachable handle or a storage handle not shown in the drawing. Furthermore, a self-adhesive sheet provided with the hydrophilic processed portion having undergone antibacterial processing may be stuck to the housing 72 having a monocoque structure as in the portable radiographic imaging device 70 shown in Fig 3, because such a sheet easily bonded.

### (Third embodiment)

### (Mammography device)

Next, a mammography device which is an instrument as a third embodiment according to the present invention will be described below.

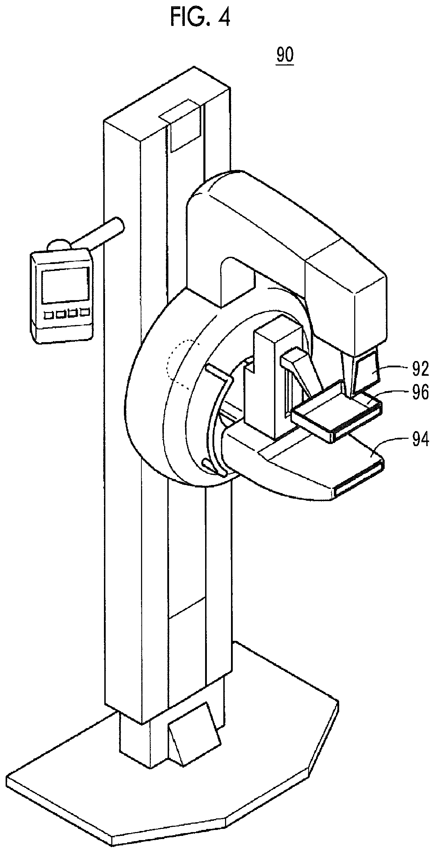

The instrument of the present invention may be a mammography device 90 shown in Fig. 4. In this case, a portion contacting a subject is an outer surface, the outer surface contains an antibacterial agent, and a hydrophilic processed portion having undergone antibacterial processing is provided. The hydrophilic processed portion can be preferably provided in, for example, a face guard 92, a breast support 94, or a breast compression plate 96.

The lipstick or sebum of a patient adheres to the face guard 92 in some cases. Moreover, oozing breast milk, blood resulting from a hemorrhage at the time of biopsy (mammotome biopsy), or sebum adheres to the breast support 94 and the breast compression plate 96 in some cases. If a hydrophilic processed portion is provided in the face guard 92, the breast support 94, or the breast compression plate 96, it is possible to remove the lipstick, breast milk, blood, and sebum simply by wiping, and to further improve a bactericidal ability compared to the related art because the wettability of a disinfectant solution is high at the time of sterilization.

Furthermore, if a hydrophilic processed portion having undergone antibacterial processing is provided, it is possible to inhibit the multiplication of bacteria remaining after wiping or to kill the bacteria.

### (Fourth embodiment)

### (Radiographic imaging device for upright radiography)

Next, a radiographic imaging device for upright radiography which is an instrument as a fourth embodiment according to the present invention will be described below.

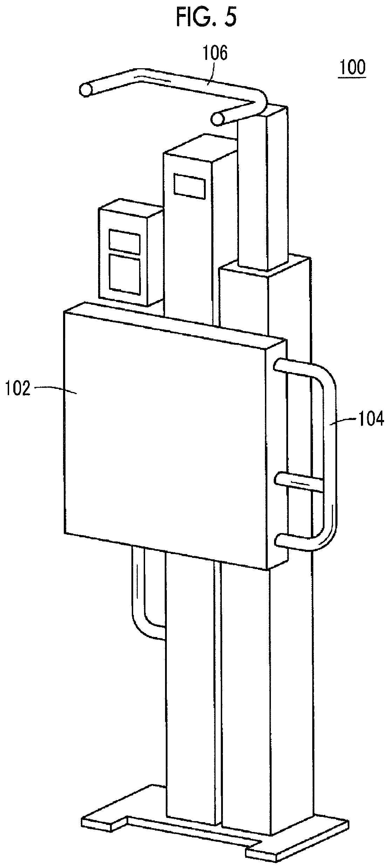

The instrument of the present invention may be a radiographic diagnostic device 100 for upright radiography shown in Fig. 5. In this case, an outer surface of an imaging board 102, which contacts a subject at the time of imaging, or an outer surface of grips 104 and 106 a subject grips at the time of imaging is preferably provided with a hydrophilic processed portion which contains an antibacterial agent and has undergone antibacterial processing. Furthermore, the hydrophilic processed portion having undergone antibacterial processing can be preferably provided on an outer surface of other portions including an operation panel portion touched by a radiographer.

### (CR Cassette)

An outer surface of a CR cassette, which accommodates an imaging plate used for computed radiography (CR) at the time of imaging, can preferably be provided with a hydrophilic processed portion which contains an antibacterial agent and has undergone antibacterial processing. In this case, similarly to the portable radiographic imaging device, it is preferable that a surface of an imaging surface contacting a subject or the entirety of the outer surface that can be touched by a radiographer is provided with such a hydrophilic processed portion.

### (Grid)

An outer surface of a grid, which is used for removing scattering radiation and improving contrast at the time of imaging performed using a portable radiographic imaging device or a CR cassette, is preferably provided with a hydrophilic processed portion which contains an antibacterial agent and has undergone antibacterial processing.

The present invention is not limited to the aforementioned examples, and an outer surface of an radiographic imaging device which contacts a subject and to which a contaminant can adhere can be provided with a hydrophilic processed portion which contains an antibacterial agent and has undergone antibacterial processing.

### (Fifth embodiment)

### (Touch panel)

Next, a touch panel which is an instrument as a fifth embodiment according to the present invention will be described below.

The instrument of the present invention may be a touch panel 112 incorporated into a display device 110 shown in Fig. 6. In this case, it is preferable that a cover member 120 of the touch panel, on which fingerprints or the like of an operator (user) such as a health professional or a patient leave, is provided with a hydrophilic processed portion 130 which contains an antibacterial agent and has undergone antibacterial processing. Furthermore, the hydrophilic processed portion having undergone antibacterial processing may be provided in other portions touched by the operator. Herein, because the hydrophilic processed portion 130 is the same as the hydrophilic processed portion described in the aforementioned first embodiment, description thereof will not be repeated.

As shown in Fig. 6, the display device 110 has a capacitance-type touch panel 112 which detects a contact position from an input surface 112a (in a direction of an arrow Z1), a display unit 114 which can display a color image and/or a monochrome image, and a housing 116 which accommodates the touch panel 112 and the display unit 114. A user can have access to the touch panel 112 through a large opening portion provided on one surface (in the direction of the arrow Z1) of the housing 116.

The touch panel 112 includes a conductive film 118 which has a mesh-like wiring layer, the cover member 120 which is laminated on one surface (in the direction of the arrow Z1) of the conductive film 118, a flexible board 124 which is electrically connected to the conductive film 118 through a cable 122, and a detection control portion 126 which is disposed on the flexible board 124.

One surface (in the direction of the arrow Z1) of the display unit 114 is bonded to the conductive film 118 through an adhesive layer 128. The conductive film 118 is disposed on a display screen, in a state where the other surface thereof (in a direction of an arrow Z2) faces the display unit 114.

By covering one surface of the conductive film 118, the cover member 120 functions as the input surface 112a. Furthermore, by preventing the direct contact of a contact object 132 (for example, a finger or a stylus pen), the cover member 120 can inhibit the occurrence of scratch, the adherence of dust, or the like and can stabilize conductivity of the conductive film 118.

The cover member 120 may be formed of a material such as glass or a resin film. In a state where one surface (in the direction of the arrow Z2) of the cover member 120 is coated with silicon oxide or the like, one surface (in the direction of the arrow Z1) of the conductive film 118 may be caused to adhere thereto. In addition, in order to prevent damage resulting from friction or the like, the conductive film 118 and the cover member 120 may be bonded to each other.

The flexible board 124 is a flexible electronic board. Although the flexible board 124 is fixed to a lateral inner wall of the housing 116 in the example shown in the drawing, the installation position of the flexible board 124 may be changed in many ways. The detection control portion 126 constitutes an electronic circuit which detects a change of capacitance between the contact object 132 and the conductive film 118 when the contact object 132 as a conductor contacts (or approaches) the input surface 112a, and detects the contact position (or approaching position).

On a surface (in the direction of the arrow Z1) of the cover member 120 that becomes the input surface 112a of the touch panel 112, the hydrophilic processed portion 130 is formed. The hydrophilic processed portion 130 may be formed on the entirety or a portion of the surface of the cover member 120 that becomes the input surface 112a. It is preferable that the hydrophilic processed portion 130 is formed on the entire region contacting the contact object 132. The contact object 132 such as a finger of a user contacts the input surface 112a of the touch panel 112, and hence a contaminant easily adheres to the input surface 112a. However, because the hydrophilic processed portion 130 is present between the contact object 132 and the surface of the cover member 120 that becomes the input surface 112a, water goes in between the contaminant and the outer surface. Therefore, the contaminant is easily removed, bacterial multiplication can be inhibited, and fingerprints of a finger of the user do not easily leave. Furthermore, visibility of the display screen of the display unit 114 does not degrade or deteriorate.

### (Sixth embodiment)

### (Protective sheet)

Next, a protective sheet as a sixth embodiment according to the present invention will be described below.

As shown in Fig. 7A, a protective sheet 140 of the present invention has a main sheet 142, a hydrophilic processed portion 144 which is formed on one external surface of the main sheet 142, an pressure sensitive adhesive layer 146 which is formed on the other surface of the main sheet 142 that is on the side opposite to one external surface, and a release sheet 148 which is laminated on a surface of the pressure sensitive adhesive layer 146 that is on the side opposite to the main sheet 142.

The protective sheet of the present invention is not limited to an embodiment in which the hydrophilic processed portion 144 is formed on the entirety of one outer surface of the main sheet 142 as in the protective sheet 140 shown in Fig. 7A. The protective sheet of the present invention may be constituted such that the hydrophilic processed portion 144 is formed on a portion of one outer surface of the main sheet 142 as in a protective sheet 141 shown in Fig. 7B.

The protective sheets 140 and 141 of the present invention are used for forming a laminate of the hydrophilic processed portion 144 and the main sheet 142, on a hydrophilic processed portion forming surface of various instruments, such as the first to fifth embodiments, used in the present invention described above.

In the examples shown in Figs. 7A and 7B, the protective sheets 140 and 141 have the pressure sensitive adhesive layer 146. Therefore, by peeling the release sheet 148 from the pressure sensitive adhesive layer 146 and bonding the pressure sensitive adhesive layer 146 to a hydrophilic processed portion forming surface of various instruments described above, for example, the irradiation surface 19 of the portable radiographic imaging device 10 shown in Fig. 1, the irradiation surface 86 of the portable radiographic imaging device 70 shown in Fig. 3, the face guard 92, the breast support 94, or the breast compression plate 96 of the mammography device 90 shown in Fig. 4, the imaging board 102 and the grips 104 and 106 of the radiographic diagnostic device 100 for upright radiography shown in Fig. 5, or the input surface 112a (surface of the cover member 120) of the touch panel 112 shown in Fig. 6, a laminate of the hydrophilic processed portion 144 and the main sheet 142 can be bonded to and mounted on the hydrophilic processed portion forming surface through the pressure sensitive adhesive layer 146.

In the examples shown in Figs. 7A and 7B, the protective sheets 140 and 141 have the pressure sensitive adhesive layer 146 in addition to the laminate of the hydrophilic processed portion 144 and the main sheet 142. However, the present invention is not limited thereto, and the protective sheets 140 and 141 may be constituted only with the laminate of the hydrophilic processed portion 144 and the main sheet 142. In a case where the protective sheets 140 and 141 are constituted only with the laminate of the hydrophilic processed portion 144 and the main sheet 142, by additionally forming an adhesive layer or the like by means of coating the hydrophilic processed portion forming surface or a surface of the main sheet 142 with an adhesive or the like, and bonding the laminate of the hydrophilic processed portion 144 and the main sheet 142 to the hydrophilic processed portion forming surface, the hydrophilic processed portion 144 can be formed.

Herein, because the hydrophilic processed portion 144 is the same as the hydrophilic processed portion described in the aforementioned first embodiment, description thereof will not be repeated.

The main sheet 142 supports the hydrophilic processed portion 144 formed on the entirety or a portion of one outer surface thereof. The hydrophilic processed portion 144 may be formed on the entirety or a portion of one outer surface of the main sheet 142. It is preferable that the hydrophilic processed portion 144 is formed on the entirety of one outer surface of the main sheet 142.

The main sheet 142 is not particularly limited as long as it can support the hydrophilic processed portion 144, and any type of sheet may be used. As the main sheet 142, a known sheet can be used. For example, it is possible to use a polyethylene terephthalate film, a polybutylene terephthalate film (PBT), a polyimide film, a triacetyl cellulose film, and the like. As PET, it is possible to use LUMIRROR U34 manufactured by TORAY INDUSTRIES, INC, COSMOSHINE A4300 manufactured by Toyobo Co., Ltd, O3916W manufactured by TEIJIN LIMITED, and the like. Furthermore, an easily adhesive layer may be provided on a surface thereof.

A thickness of the main sheet 142 is not particularly limited, and those having a thickness of 10 µm to 200 µm can be used. In a case where the laminate of the hydrophilic processed portion 144 and the main sheet 142 is to be bonded to a resistive film-type touch panel, the laminate needs to conform to a flexible surface, and hence the thickness of the main sheet 142 is 10 µm to 100 µm and preferably 10 µm to 50 µm. In a case of a capacitance-type touch panel, in view of ease of bonding, it is possible to preferably use a main sheet 142 having a thickness of 50 µm to 100 µm.

The pressure sensitive adhesive layer 146 is used for bonding the laminate of the hydrophilic processed portion 144 and the main sheet 142 to the hydrophilic processed portion forming surface of the various instruments described above. The pressure sensitive adhesive layer 146 is not particularly limited as long as it enables the laminate of the hydrophilic processed portion 144 and the main sheet 142 to be bonded to various hydrophilic processed portion forming surfaces, and may be formed using a known pressure sensitive adhesive. The pressure sensitive adhesive usable in the pressure sensitive adhesive layer 146 is not particularly limited, and examples thereof include a (meth)acrylic pressure sensitive adhesive, a rubber-based pressure sensitive adhesive, a silicone-based pressure sensitive adhesive, a urethane-based pressure sensitive adhesive, a polyester-based pressure sensitive adhesive, and the like. In a case where the pressure sensitive adhesive layer is used for a surface of a touch panel, considering the facts that the pressure sensitive adhesive layer is repeatedly bonded and peeled and needs to be bonded while preventing air bubbles from entering, it is also possible to preferably use a self-adhesive pressure sensitive adhesive. Herein, the (meth)acrylic pressure sensitive adhesive refers to an acrylic pressure sensitive adhesive and/or a methacrylic pressure sensitive adhesive. As the (meth)acrylic pressure sensitive adhesive, it is possible to use a (meth)acrylic pressure sensitive adhesive used in a pressure sensitive sheet which will be described later.

A method for forming a pressure sensitive adhesive layer is not particularly limited, and examples thereof include a coating method, a printing method, a bonding method, and the like. Among these, it is possible to preferably use a method of installing the pressure sensitive adhesive layer by coating and a method of forming the pressure sensitive adhesive layer by bonding a pressure sensitive sheet, and the method of forming the pressure sensitive adhesive layer by bonding a pressure sensitive sheet is more preferable.

A thickness of the pressure sensitive adhesive layer 146 is not particularly limited, and is preferably 1 µm to 30 µm. If the thickness of the pressure sensitive adhesive layer is equal to or greater than 1 µm, the film can be stably formed by co-extrusion. If the thickness is equal to or less than 30 µm, costs of the material are reduced. For enhancing adhesion, it is preferable to increase the thickness of the pressure sensitive adhesive layer in consideration of the viscosity thereof, because a contact area between the pressure sensitive adhesive layer and an object covered with the pressure sensitive adhesive layer is easily increased if the thickness of the pressure sensitive adhesive layer is increased. The thickness of the pressure sensitive adhesive layer is preferably 2 µm to 20 µm, and more preferably 3 µm to 15 µm.

The adhesion of the pressure sensitive adhesive layer 146 is not particularly limited, and is preferably within a range of 2 cN/25 mm to 20 cN/25 mm for use. If the adhesion is equal to or greater than 2 cN/25 mm, when the pressure sensitive adhesive layer is used by being bonded to a surface of a touch panel or the like, the pressure sensitive adhesive layer is not easily detached. If the adhesion is equal to or less than 20 cN/25 mm, the film can be smoothly peeled off at the time of peeling.

The release sheet 148 remains bonded to the pressure sensitive adhesive layer 146 until the protective sheet 140 is used so as to protect the pressure sensitive adhesive layer 146. The release sheet 148 is not particularly limited as long as it can protect the pressure sensitive adhesive layer 146, and a known release sheet 148 can be used. For example, it is possible to use a release agent such as a silicone-based compound, a long-chain alkyl-based compound, or polyvinyl alcohol·carbamate.

A thickness of the release sheet 148 is not particularly limited, and is preferably 1 µm to 30 µm. If the thickness of the release sheet is equal to or greater than 1 µm, the film can be stably formed by co-extrusion. If the thickness is equal to or less than 30 µm, costs of the material are reduced. The thickness of the release sheet is preferably 2 µm to 20 µm, and more preferably 3 µm to 15 µm.

### (Seventh embodiment)

### (Antibacterial film)

Next, a substrate with an antibacterial film in which an antibacterial film as a seventh embodiment according to the present invention will be described below.

A substrate with an antibacterial film 150 shown in Fig. 8A has a substrate 152 and a hydrophilic portion 154 which is formed on one external surface (upper surface in the example illustrated in the drawing) of the substrate 152. The hydrophilic portion 154 constitutes an antibacterial film 156 of the present invention.

The antibacterial film 156 of the present invention is not limited to the antibacterial film shown in Fig. 8A that is constituted with the hydrophilic portion 154 formed on the entirety of one outer surface of the substrate 152. As a substrate with an antibacterial film 150A shown in Fig. 8B, the antibacterial film 156 may be formed on the entirety of one outer surface of the substrate 152 so as to include the hydrophilic portion 154 formed on a portion of one outer surface of the substrate 152. Alternatively, as a substrate with an antibacterial film 150B shown in Fig. 8C, the antibacterial film 156 may be constituted with the hydrophilic portion 154 formed on a portion of one outer surface of the substrate 152.

Examples of the substrate 152 of the substrates with an antibacterial film 150, 150A, and 150B shown in Figs. 8A, 8B, and 8C include members constituting the hydrophilic processed portion forming surface of various instruments described above, such as members of medical instruments including the top panel 64 and the holding portion 66 constituting the irradiation surface 19 of the portable radiographic imaging device 10 shown in Figs. 1 and 2, the body member 76 constituting the irradiation surface 86 of the portable radiographic imaging device 70 shown in Fig. 3, the face guard 92, the breast support 94, or the breast compression plate 96 of the mammography device 90 shown in Fig. 4, and the imaging board 102 and the grips 104 and 106 of the radiographic diagnostic device 100 for upright radiography shown in Fig. 5, and the cover member 120 constituting the input surface 112a of the touch panel 112 shown in Fig. 6. Examples of the substrate 152 also include a protective sheet to be stuck to a member constituting the hydrophilic processed portion forming surface of these instruments, such as the protective sheets 140 and 141 and the main sheet 142 shown in Figs. 7A and 7B.

In the present invention, the substrate 152 is not limited to the above. It goes without saying that the substrate 152 can be applied to members having an external surface which needs to have antibacterial properties because the surface is touched by a human being, such as ceramic sanitary wares such as toilets and toilet seats, handles of various members (metal or resin), walls, doors, window glass, and the like of houses, furniture such as tables and dining tables, and home appliances such as refrigerators, cooking utensils, cleaners, washing machines, audio devices, and TV.

The hydrophilic portion 154 of the substrates with an antibacterial film 150, 150A, and 150B shown in Figs. 8A, 8B, and 8C is disposed on at least a portion of a surface of the substrate 152. Specifically, the hydrophilic portion 154 may be disposed on the entirety of one surface of the substrate 152 as the substrate with an antibacterial film 150 shown in Fig. 8A, or may be disposed only on a portion of a surface of the substrate 152 as the substrates with an antibacterial film 150A and 150B shown in Figs. 8A and 8C. Furthermore, the substrate 152 may constitute the entirety of the antibacterial film 156 as the substrates with an antibacterial film 150 and 150B shown in Figs. 8A and 8C, or may constitute only a portion of the antibacterial film 156 as the substrate with an antibacterial film 150A shown in Fig. 8B.

Examples of the hydrophilic portion 154 include the hydrophilic processed portion of the present invention, specifically, the hydrophilic processed portion forming the outer surface of the aforementioned members or main sheet, such as members of medical instruments including the hydrophilic processed portion 20 forming the irradiation surface 19 of the portable radiographic imaging device 10 shown in Figs. 1 and 2 and the hydrophilic processed portion 87 forming the irradiation surface 86 of the portable radiographic imaging device 70 shown in Fig. 3, and the hydrophilic processed portion 130 forming the input surface 112a of the touch panel 112 shown in Fig. 6. In addition, it can be mentioned that the constitution of the hydrophilic portion 154 is exactly the same as the constitution of, for example, the hydrophilic processed portion 144 of the protective sheets 140 and 141 shown in Figs. 7A and 7B.

That is, the constitution of the hydrophilic portion of the present invention is exactly the same as the constitution of the hydrophilic processed portion of the present invention described above. Therefore, the constitution of the hydrophilic portion will not be described.

The antibacterial film 156 of the substrates with an antibacterial film 150, 150A, and 150B shown in Figs. 8A, 8B, and 8C is a film which is disposed on at least a portion of a surface of the substrate 152 and has an antibacterial activity, and at least a portion of the antibacterial film 156 is hydrophilic. That is, at least a portion of the antibacterial film 156 is constituted with the hydrophilic portion 154.

Specifically, the antibacterial film 156 may be disposed on the entirety of one surface of the substrate 152 as the substrates with an antibacterial film 150 and 150A shown in Figs. 8A and 8B, or may be disposed only on a portion of a surface of the substrate 152 as the substrate with an antibacterial film 150B shown in Fig. 8C. Furthermore, the entirety of the antibacterial film 156 may be constituted with the hydrophilic portion 154 as the substrates with an antibacterial film 150 and 150B shown in Figs. 8A and 8C. Alternatively, only a portion of the antibacterial film 156 may be constituted with the hydrophilic portion 154 as the substrate with an antibacterial film 150A shown in Fig. 8B.

The antibacterial film contains at least one kind of silver-containing antibacterial agent. The silver-containing antibacterial agent (silver-based antibacterial agent) is the same as the aforementioned silver-based antibacterial agent, and hence the description thereof will not be repeated.

In the present invention, a content of the silver-based antibacterial agent in the antibacterial film is not particularly limited as the content of the silver-based antibacterial agent in the hydrophilic processed portion. In view of further improving effects of the present invention, it is preferable that the silver-based antibacterial agent is incorporated into the antibacterial film such that a content of silver with respect to a total mass of the antibacterial film becomes 0.001 to 20 wt% (preferably 0.001 to 10 wt%, and more preferably 0.001 to 5 wt%).

In a case where an organic antibacterial agent is used as a silver-based antibacterial agent, a content of the antibacterial agent is not particularly limited. In view of further improving mechanical strength of the antibacterial film and improving effects of the present invention, the content of the antibacterial agent is preferably 1 to 4% by mass with respect to a total mass of the antibacterial film.

In a case where an inorganic antibacterial agent is used as a silver-based antibacterial agent, a content of the antibacterial agent is not particularly limited. In view of further improving mechanical strength of the antibacterial film and improving effects of the present invention, the content of the antibacterial agent is preferably 0.001 to 10 wt% and more preferably 0.01 to 5 wt% with respect to a total mass of the antibacterial film.

### (Substrate with antibacterial film)

It can be mentioned that the aforementioned substrate with an antibacterial film including a substrate and an antibacterial film is a substrate with a hydrophilic portion. In the substrate with an antibacterial film, an amount of silver ions per unit area measured by the aforementioned extraction test is preferably equal to or greater than 15 ng/cm², more preferably 15 ng/cm² to 100 ng/cm², even more preferably 15 ng/cm² to 75 ng/cm², and particularly preferably 15 ng/cm² to 50 ng/cm² as in the aforementioned substrate with a hydrophilic processed portion, for the same reason as described above.

In the example described above, the antibacterial film is used by being formed on a substrate, but the present invention is not limited thereto. It goes without saying that the antibacterial film can be used alone.

### (Method for manufacturing antibacterial film)

A method for manufacturing an antibacterial film layer is not particularly limited, and a known method can be adopted. Examples of the method include coating methods such as a screen printing method, a dip coating method, a spray coating method, a spin coating method, an ink jet method, and a bar coating method.

### [Examples]

### [Example 1]

In the protective sheet 140 shown in Fig. 7A, a PET base (COSMOSHINE A4300 (manufactured by Toyobo Co., Ltd), thickness: 50 µm) having an easily adhesive layer on a surface which will become the main sheet 142 was coated with a coating agent containing a composition for forming a hydrophilic processed portion (hereinafter, referred to as a composition for forming an antibacterial coating film) containing an antibacterial agent which will be described later, and the coating agent was cured (treated by being irradiated with ultraviolet rays), thereby providing an antibacterial coating film which will become the hydrophilic processed portion 144. An average thickness of the antibacterial coating film was about 2 µm.

A strongly adhesive side of a mount-type GELPOLY sheet (manufactured by PANAC Co., Ltd.) was bonded to a rear surface of the PET base, thereby obtaining a protective sheet of Example 1 as a film with a pressure sensitive adhesive layer. The GEL POLY sheet had a property of making it difficult for air bubbles to enter the sheet even when the sheet is bonded to a surface of glass or the like. The GEL POLY sheet was suitable for the protective sheet to be bonded to a surface of a touch panel or the like.

### (Composition for forming antibacterial coating film)

The composition contained the following components.

| | |
|---|---|
| Monomer having hydrophilic group: | |
| Miramer M4004 (manufactured by Toyo Chemicals Co., Ltd.) | 37 parts by mass |
| Monomer having hydrophilic group: | |
| Miramer M3150 (manufactured by Toyo Chemicals Co., Ltd.) | 37 parts by mass |
| Antibacterial agent: silver ceramic particle dispersion (manufactured by Fuji Chemical Industries, Ltd., average particle size: 0.8 µm, concentration of antibacterial agent: 50 wt%) | 1 part by mass |
| Cross-linking agent: A-DPH (manufactured by SHIN-NAKAMURA CHEMICAL CO., LTD.) | 22 parts by mass |
| Polymerization initiator: IRGACURE 184 (manufactured by BASF SE) | 3 parts by mass |
| Solvent: methoxypropanol | 150 parts by mass |

Because the solvent volatilizes, the mass of the components other than the solvent is the mass of the hydrophilic processed portion. The same will be applied in the following examples.

### [Example 2]

A protective sheet of Example 2 was prepared in the same manner as in Example 1, except that, in Example 1, monomers having a hydrophilic group as components of the composition for forming an antibacterial coating film were changed as below.

Herein, the monomer Miramer M420 does not contain a hydrophilic group.

| | |
|---|---|
| Monomer having hydrophilic group: | |
| Miramer M4004 (manufactured by Toyo Chemicals Co., Ltd.) | 34 parts by mass |
| Monomer having hydrophilic group: | |
| Mirmaer M3150 (manufactured by Toyo Chemicals Co., Ltd.) | 34 parts by mass |
| Monomer: | |
| Miramer M420 (manufactured by Toyo Chemicals Co., Ltd.) | 6 parts by mass |

### [Example 3]

A protective sheet of Example 3 was prepared in the same manner as in Example 2, except that, in Example 2, monomers having a hydrophilic group and a monomer as components of composition for forming an antibacterial coating film were formulated with each other in an amount changed as below.

| | |
|---|---|
| Monomer having hydrophilic group: | |
| Miramer M4004 (manufactured by Toyo Chemicals Co., Ltd.) | 32 parts by mass |
| Monomer having hydrophilic group: | |
| Mirmaer M3150 (manufactured by Toyo Chemicals Co., Ltd.) | 32 parts by mass |
| Monomer: | |
| Miramer M420 (manufactured by Toyo Chemicals Co., Ltd.) | 10 parts by mass |

### [Example 4]

A protective sheet of Example 4 was prepared in the same manner as in Example 2, except that, in Example 2, monomers having a hydrophilic group and a monomer as components of composition for forming an antibacterial coating film were formulated with each other in an amount changed as below.

| | |
|---|---|
| Monomer having hydrophilic group: | |
| Miramer M4004 (manufactured by Toyo Chemicals Co., Ltd.) | 30 parts by mass |
| Monomer having hydrophilic group: | |
| Mirmaer M3150 (manufactured by Toyo Chemicals Co., Ltd.) | 30 parts by mass |
| Monomer: | |
| Miramer M420 (manufactured by Toyo Chemicals Co., Ltd.) | 14 parts by mass |

### [Example 5]

A protective sheet of Example 5 was prepared in the same manner as in Example 4, except that, in Example 4, a monomer and an antibacterial agent were formulated with each other in an amount changed as below.

| | |
|---|---|
| Monomer: | |
| Miramer M420 (manufactured by Toyo Chemicals Co., Ltd.) | 13 parts by mass |
| Antibacterial agent: silver ceramic particle dispersion (manufactured by Fuji Chemical Industries, Ltd., average particle size: 0.8 µm, concentration of antibacterial agent: 50 wt%) | 2 parts by mass |

### [Example 6]

A protective sheet of Example 6 was prepared in the same manner as in Example 4, except that, in Example 4, a monomer and an antibacterial agent were formulated with each other in an amount changed as below.

| | |
|---|---|
| Monomer: | |
| Miramer M420 (manufactured by Toyo Chemicals Co., Ltd.) | 12 parts by mass |
| Antibacterial agent: silver ceramic particle dispersion (manufactured by Fuji Chemical Industries, Ltd., average particle size: 0.8 µm, concentration of antibacterial agent: 50 wt%) | 3 parts by mass |

### [Example 7]

A protective sheet of Example 7 was prepared in the same manner as in Example 4, except that, in Example 4, a monomer and an antibacterial agent were formulated with each other in an amount changed as below.

| | |
|---|---|
| Monomer: | |
| Miramer M420 (manufactured by Toyo Chemicals Co., Ltd.) | 10 parts by mass |
| Antibacterial agent: silver ceramic particle dispersion (manufactured by Fuji Chemical Industries, Ltd., average particle size: 0.8 µm, concentration of antibacterial agent: 50 wt%) | 5 parts by mass |

### [Example 8]

A protective sheet of Example 8 was prepared in the same manner as in Example 7, except that, in Example 7, monomers having a hydrophilic group and a monomer as components of the composition for forming an antibacterial coating film were formulated with each other in an amount changed as below.

| | |
|---|---|
| Monomer having hydrophilic group: | |
| Miramer M4004 (manufactured by Toyo Chemicals Co., Ltd.) | 25 parts by mass |
| Monomer having hydrophilic group: | |
| Mirmaer M3150 (manufactured by Toyo Chemicals Co., Ltd.) | 25 parts by mass |
| Monomer: | |
| Miramer M420 (manufactured by Toyo Chemicals Co., Ltd.) | 24 parts by mass |

### [Example 9]

A protective sheet of Example 9 was prepared in the same manner as in Example 1, except that, in Example 1, monomers having a hydrophilic group as components of the composition for forming an antibacterial coating film were changed as below.

| | |
|---|---|
| Monomer having hydrophilic group: | |
| Miramer M4004 (manufactured by Toyo Chemicals Co., Ltd.) | 34 parts by mass |
| Monomer having hydrophilic group: | |
| Mirmaer M3150 (manufactured by Toyo Chemicals Co., Ltd.) | 34 parts by mass |
| Monomer: | |
| Miramer M420 (manufactured by Toyo Chemicals Co., Ltd.) | 2 parts by mass |
| Hydrophilic polymer: PEG 200 (manufactured by KANTO CHEMICAL CO.,INC.) | 4 parts by mass |

### [Example 10]

A protective sheet of Example 10 was prepared in the same manner as in Example 9, except that, in Example 9, each of the materials was formulated in an amount changed as below.

| | |
|---|---|
| Monomer having hydrophilic group: | |
| Miramer M4004 (manufactured by Toyo Chemicals Co., Ltd.) | 32 parts by mass |
| Monomer having hydrophilic group: | |
| Miramer M3150 (manufactured by Toyo Chemicals Co., Ltd.) | 32 parts by mass |
| Monomer: | |
| Miramer M420 (manufactured by Toyo Chemicals Co., Ltd.) | 2 parts by mass |
| Hydrophilic polymer: PEG 200 (manufactured by KANTO CHEMICAL CO.,INC.) | 8 parts by mass |

### [Example 11]

A protective sheet of Example 11 was prepared in the same manner as in Example 1, except that, in Example 1, the following silver particle dispersion was added to the composition for forming an antibacterial coating film.

| | |
|---|---|
| Antibacterial agent: silver particle dispersion (manufactured by Japan Ion Corporation, particle size: about 7 to 10 nm, concentration of silver particles: 1 wt%) | 1 part by mass |

### [Example 12]

A protective sheet of Example 12 was prepared in the same manner as in Example 10, except that, in Example 10, the following silver particle dispersion was added to the composition for forming an antibacterial coating film.

| | |
|---|---|
| Antibacterial agent: silver particle dispersion (manufactured by Japan Ion Corporation, particle size: about 7 to 10 nm, concentration of silver particles: 1 wt%) | 1 part by mass |

### [Example 13]

A protective sheet of Example 13 was prepared in the same manner as in Example 2, except that, in Example 2, each of the materials was formulated in an amount changed as below.

| | |
|---|---|
| Monomer having hydrophilic group: | |
| Miramer M4004 (manufactured by Toyo Chemicals Co., Ltd.) | 10 parts by mass |
| Monomer having hydrophilic group: | |
| Mirmaer M3150 (manufactured by Toyo Chemicals Co., Ltd.) | 10 parts by mass |
| Monomer: | |
| Miramer M420 (manufactured by Toyo Chemicals Co., Ltd.) | 54 parts by mass |

### [Example 14]

A protective sheet of Example 14 was prepared in the same manner as in Example 9, except that, in Example 9, each of the materials was formulated in an amount changed as below.

| | |
|---|---|
| Monomer having hydrophilic group: | |
| Miramer M4004 (manufactured by Toyo Chemicals Co., Ltd.) | 30 parts by mass |
| Monomer having hydrophilic group: | |
| Mirmaer M3150 (manufactured by Toyo Chemicals Co., Ltd.) | 30 parts by mass |
| Hydrophilic polymer: PEG 200 (manufactured by KANTO CHEMICAL CO.,INC.) | 14 parts by mass |

### [Example 15]

A protective sheet of Example 15 was prepared in the same manner as in Example 1, except that, in Example 1, monomers having a hydrophilic group and a monomer as components of the composition for forming an antibacterial coating film were formulated with each other in an amount changed as below, and a component as an antibacterial agent was changed as below. T/Da equaled 100.

| | |
|---|---|
| Monomer having hydrophilic group: | |
| Miramer M4004 (manufactured by Toyo Chemicals Co., Ltd.) | 28 parts by mass |
| Monomer having hydrophilic group: | |
| Mirmaer M3150 (manufactured by Toyo Chemicals Co., Ltd.) | 28 parts by mass |
| Monomer: | |
| Miramer M420 (manufactured by Toyo Chemicals Co., Ltd.) | 17 parts by mass |
| Antibacterial agent: mixed dispersion of silver ceramic particles and titanium oxide particles (0.55 parts by mass of titanium oxide particles (manufactured by NIPPON AEROSIL CO., LTD., average particle size: 20 nm) was added to and mixed with 0.9 parts by mass of silver ceramic particle dispersion (manufactured by Fuji Chemical Industries, Ltd., average particle size: 0.8 µm, concentration of antibacterial agent: 50 wt%)) | 1.45 parts by mass |

### [Example 16]

A protective sheet of Example 16 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.4 parts by mass of titanium oxide particles (manufactured by NIPPON AEROSIL CO., LTD., average particle size: 20 nm) was added to and mixed with 1.2 parts by mass of silver ceramic particle dispersion (manufactured by Fuji Chemical Industries, Ltd., average particle size: 0.8 µm, concentration of antibacterial agent: 50 wt%)) | 1.6 parts by mass |

### [Example 17]

A protective sheet of Example 17 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.7 parts by mass of titanium oxide particles (manufactured by NIPPON AEROSIL CO., LTD., average particle size: 20 nm) was added to and mixed with 0.6 parts by mass of silver ceramic particle dispersion (manufactured by Fuji Chemical Industries, Ltd., average particle size: 0.8 µm, concentration of antibacterial agent: 50 wt%)) | 1.3 parts by mass |

### [Example 18]

A protective sheet of Example 18 was prepared in the same manner as in Example 15, except that, in Example 15, the amount of the antibacterial agent component formulated was changed as below.

| | |
|---|---|
| Antibacterial agent: mixed dispersion of silver ceramic particles and 3 titanium oxide particles (1.25 parts by mass of titanium oxide particles (manufactured by NIPPON AEROSIL CO., LTD., average particle size: 20 nm) was added to and mixed with 2.5 parts by mass of silver ceramic particle dispersion (manufactured by Fuji Chemical Industries, Ltd., average particle size: 0.8 µm, concentration of antibacterial agent: 50 wt%)) | 3.75 parts by mass |

### [Example 19]

A protective sheet of Example 19 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.25 parts by mass of titanium oxide particles (manufactured by NIPPON AEROSIL CO., LTD., average particle size: 20 nm) was added to and mixed with 1.5 parts by mass of silver ceramic particle dispersion (manufactured by Fuji Chemical Industries, Ltd., average particle size: 0.8 µm, concentration of antibacterial agent: 50 wt%)) | 1.75 parts by mass |

### [Example 20]

A protective sheet of Example 20 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.19 parts by mass of titanium oxide particles (manufactured by NIPPON AEROSIL CO., LTD., average particle size: 20 nm) was added to and mixed with 0.12 parts by mass of silver ceramic particle dispersion (manufactured by Fuji Chemical Industries, Ltd., average particle size: 0.8 µm, concentration of antibacterial agent: 50 wt%)) | 0.31 parts by mass |

### [Example 21]

A protective sheet of Example 21 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.375 parts by mass of titanium oxide particles (manufactured by NIPPON AEROSIL CO., LTD., average particle size: 20 nm) was added to and mixed with 0.75 parts by mass of silver ceramic particle dispersion (manufactured by Fuji Chemical Industries, Ltd., average particle size: 0.8 µm, concentration of antibacterial agent: 50 wt%)) | 1.125 parts by mass |

### [Example 22]

A protective sheet of Example 22 was prepared in the same manner as in Example 15, except that, in Example 15, the antibacterial agent component and the amount of the antibacterial agent component formulated were changed as below. T/Da equaled 2.86.

| | |
|---|---|
| Antibacterial agent: mixed dispersion of silver ceramic particles and tungsten oxide particles (0.355 parts by mass of tungsten oxide particles (manufactured by A.L.M.T. Corp., average particle size: 0.7 µm) was added to and mixed with 2.3 parts by mass of silver ceramic particle dispersion (manufactured by Fuji Chemical Industries, Ltd., average particle size: 0.8 µm, concentration of antibacterial agent: 50 wt%) | 2.65 parts by mass |

### [Example 23]

A protective sheet of Example 23 was prepared in the same manner as in Example 22, except that, in Example 22, the amount of an antibacterial agent component formulated was changed as below.

| | |
|---|---|
| Antibacterial agent: mixed dispersion of silver ceramic particles and tungsten oxide particles (0.18 parts by mass of tungsten oxide particles (manufactured by A.L.M.T. Corp., average particle size: 0.7 µm) was added to and mixed with 0.64 parts by mass of silver ceramic particle dispersion (manufactured by Fuji Chemical Industries, Ltd., average particle size: 0.8 µm, concentration of antibacterial agent: 50 wt%)) | 0.82 parts by mass |

### [Example 24]

A protective sheet of Example 24 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.22 parts by mass of titanium oxide particles (manufactured by NIPPON AEROSIL CO., LTD., average particle size: 20 nm) was added to and mixed with 1.56 parts by mass of silver ceramic particle dispersion (manufactured by Fuji Chemical Industries, Ltd., average particle size: 0.8 µm, concentration of antibacterial agent: 50 wt%)) | 1.78 parts by mass |

### [Example 25]

A protective sheet of Example 25 was prepared in the same manner as in Example 15, except that, in Example 15, the amount of the antibacterial agent component formulated was changed as below.

| | |
|---|---|
| Antibacterial agent: mixed dispersion of silver ceramic particles and titanium oxide particles (0.835 parts by mass of titanium oxide particles (manufactured by NIPPON AEROSIL CO., LTD., average particle size: 20 nm) was added to and mixed with 0.33 parts by mass of silver ceramic particle dispersion (manufactured by Fuji Chemical Industries, Ltd., average particle size: 0.8 µm, concentration of antibacterial agent: 50 wt%)) | 1.165 parts by mass |

### [Example 26]

A protective sheet of Example 26 was prepared in the same manner as in Example 15, except that, in Example 15, the antibacterial agent component and the amount of the antibacterial agent formulated were changed as below. T/Da equaled 200.

| | |
|---|---|
| Antibacterial agent: mixed dispersion of silver particles and titanium oxide particles (0.017 parts by mass of titanium oxide particles (manufactured by NIPPON AEROSIL CO., LTD., average particle size: 20 nm) was added to and mixed with 0.33 parts by mass of silver particle dispersion (manufactured by Japan Ion Corporation, average particle size: about 7 to 10 nm, concentration of silver particles: 1 wt%)) | 0.347 parts by mass |

### [Example 27]

A protective sheet of Example 27 was prepared in the same manner as in Example 26, except that, in Example 26, the amount of the antibacterial agent component formulated was changed as below.

| | |
|---|---|
| Antibacterial agent: mixed dispersion of silver particles and titanium oxide particles (0.003 parts by mass of titanium oxide particles (manufactured by NIPPON AEROSIL CO., LTD., average particle size: 20 nm) was added to and mixed with 0.67 parts by mass of silver particle dispersion (manufactured by Japan Ion Corporation, average particle size: about 7 to 10 nm, concentration of silver particles: 1 wt%)) | 0.673 parts by mass |

### [Example 28]

A protective sheet of Example 28 was prepared in the same manner as in Example 15, except that, in Example 15, the antibacterial agent component and the amount of the antibacterial agent component formulated were changed as below. T/Da equaled 2.86.

| | |
|---|---|
| Antibacterial agent: mixed dispersion of silver particles and tungsten oxide particles (0.003 parts by mass of tungsten oxide particles (manufactured by A.L.M.T. Corp., average particle size: 0.7 µm) was added to and mixed with 0.67 parts by mass of silver particle dispersion (manufactured by Japan Ion Corporation, average particle size: about 7 to 10 nm, concentration of silver particles: 1 wt%)) | 0.673 parts by mass |

### [Comparative Example 1]

A protective sheet of Comparative Example 1 was prepared by providing a hydrophilic processed portion according to the same procedure as in Example 1, except that, in Example 1, a monomer Miramer M420 not containing a hydrophilic group was used instead of a monomer having a hydrophilic group as a component of the composition for forming an antibacterial coating film.

### [Comparative Example 2]

A glass plate with an antibacterial coating film (antibacterial film) of Comparative Example 2 was prepared in the following manner according to Example 1 of JP2008-213206A.

The following components (except for a silver particle dispersion) were mixed together and stirred for 4 hours at 20°C. Then, 0.004 g of silver particle dispersion (aqueous solution containing 22.5 wt% of silver, manufactured by Sumitomo Electric Industries, Ltd.) was added to the mixture, followed by stirring for 30 minutes at 20°C, thereby preparing a solution for formation.

| | |
|---|---|
| •Ethyl alcohol (manufactured by KATAYAMA CHEMICAL, LTD.) | 5.74 g |
| •Tetraethoxysilane (manufactured by Shin-Etsu Chemical Co., Ltd.) | 1.91 g |
| •Pure water | 2.12 g |
| •p-toluene sulfonic acid monohydrate (manufactured by KATAYAMA CHEMICAL, LTD.) | 0.003 g |
| •Isobutyl alcohol (manufactured by KANTO CHEMICAL CO.,INC.) | 0.11 g |
| •Polyether phosphoric acid ester-based surfactant (SOLSPERSE 41000 manufactured by Lubrizol Japan Limited) | 0.1 g |
| •Polyethylene glycol 200 (PEG 200) (manufactured by KANTO CHEMICAL CO.,INC.) | 0.014 g |
| •Silver dispersion: aqueous solution containing 22.5 wt% of silver particles (manufactured by Sumitomo Electric Industries, Ltd.) | 0.004 g |

Then, a washed soda lime silicate glass board (100 × 100 mm, thickness: 3.1 mm, hereinafter, referred to as a "glass plate") was coated with the solution for formation by a flow coating method at a relative humidity of 30% at room temperature. The solution for formation with which the glass plate was coated was air-dried for about 15 minutes at room temperature and then heated for 20 minutes by being put into an oven preheated to 200°C, following by cooling, thereby preparing a glass plate with an antibacterial coating film of Comparative Example 2.

For the protective sheets of Examples 1 to 28 and Comparative Example 1 and the antibacterial coating film of the glass plate with an antibacterial coating film of Comparative Example 2 that were prepared as above, a contact angle, an amount of silver ions, a Vickers hardness, an water absorption rate, and a pencil hardness were measured, and antibacterial properties and hard coat properties thereof were evaluated. The results are summarized in Table 1. Table 1 also shows the makeup of the composition for forming an antibacterial coating film of the protective sheets of Examples 1 to 28 and Comparative Example 1.

### <Various measurements>

### (Contact angle)

By using LSE-ME1 (software 2win mini) (manufactured by NiCK Corporation), 2 µl of droplets of pure water were dropped onto the surface of the antibacterial coating films of Examples 1 to 28 and Comparative Examples 1 and 2 kept horizontal at room temperature of 20°C based on a sessile drop method of JIS R 3257:1999, and a contact angle at a point in time when 20 seconds had elapsed from the dropping was measured.

### (Amount of silver (Ag) ions)

By performing the same extraction test as the extraction test described above on each of the antibacterial coating films (hydrophilic processed portions), an amount (extracted amount) of silver ions per unit area of the antibacterial coating films (hydrophilic processed portions) of Examples 1 to 28 and Comparative Examples 1 and 2 was calculated by the same calculation method as the calculation method described above.

### (Vickers hardness)

A Vickers hardness of each of the antibacterial coating films (hydrophilic processed portions) of Examples 1 to 28 and Comparative Examples 1 and 2 was measured in a manner described above by using a Vickers hardness tester (manufactured by Fischer Instruments K.K.) under a load applied at 20 mN/20 s for a loading time of 5 s.

### (Water absorption rate)

A water absorption rate of each of the antibacterial coating films (hydrophilic processed portions) of Examples 1 to 28 and Comparative Examples 1 and 2 was measured by the same measurement method as the aforementioned method for measuring a water absorption rate of a hydrophilic processed portion.

### <Various evaluations>

### (Antibacterial properties)

By using *E. coli* as a bacterial strain, each of the examples and comparative examples was tested for each time period by being brought into contact with the bacteria for not longer than 24 hours based on JIS Z 2801, and the time taken for the number of living bacteria to become equal to or less than a limit of detection was measured.

Based on the time taken for the number of living bacteria to become equal to or less than a limit of detection measured as above, antibacterial properties were evaluated.

In a case where the time taken for the number of living bacteria to become equal to or less than a limit of detection was equal to or less than 30 minutes, the example was evaluated to be A "antibacterial properties are quite excellent". In a case where the time was longer than 30 minutes and equal to or less than 60 minutes, the example was evaluated to be B "antibacterial properties are excellent". In a case where the time was longer than 60 minutes and equal to or less than 2 hours, the example was evaluated to be C "antibacterial properties are fair". In a case where the time was longer than 2 hours and equal to or less than 3 hours, the example was evaluated to be D "antibacterial properties are poor". In a case where the time was longer than 3 hours, the example was evaluated to be E "antibacterial properties are very poor".

The results of evaluation of antibacterial properties are summarized in Table 1.

### (Hard coat properties)

According to a pencil hardness testing method of JIS K 5600-5-4, a pencil hardness test was performed, and scratch hardness of the surface of each of the antibacterial coating films of Examples 1 to 28 and Comparative Examples 1 and 2 was measured.

Based on the pencil hardness measured as above, hard coat properties were evaluated.

In a case where the pencil hardness was equal to or greater than 2H, the example was evaluate to be A "hard coat properties are excellent". In a case where the pencil hardness was H or F, the example was evaluated to be B "hard coat properties are fair". In a case where the pencil hardness is smaller than F, the example was evaluated to be C "hard coat properties are poor".

The results of evaluation of hard coat properties are summarized in Table 1.

In the following Table 1, the column of "added amount" of the column of "antibacterial agent" shows an amount (part by mass) of dispersion, which was used in each example and Comparative Example 1, added. Here, the column of "added amount" of the column of "antibacterial agent" of Comparative Example 2 shows that a content of an antibacterial agent (Ag particles) is 0.2 parts by mass with respect to 100 parts by mass of a hydrophilic processed portion.

**[Table 1]**

| Table 1 (1-1) | Hydrophilic monomer (1) M4004 (part by mass) | Hydrophilic monomer (2) M3150 (part by mass) | Cross-linking agent A-DPH (part by mass) | Monomer M420 (part by mass) | Hydrophilic polymer PEG200 (part by mass) | Polymerization initiator IRGACURE 184 (part by mass) | Antibacterial agent | | Contact angle | Time taken for number of living bacterial to become equal to or less than limit of detection | Antibacterial properties | Amount of Ag ions (ng/cm²) | Water absorption rate (wt%) | Pencil hardness | Hard coat properties | Vickers hardness (HV) |
|---|---|---|---|---|---|---|---|---|---|---|---|---|---|---|---|---|
| | | | | | | | Type | Added amount (Part by mass) | | | | | (With respect to weight of antibacteria1 coat) | | | |
| Example 1 | 37 | 37 | 22 | - | - | 3 | Ag ceramic particles | 1 | 8° | Equal to or less than 30 minutes | A | 25 | 3 | 2H | A | 10 |
| Example 2 | 34 | 34 | 22 | 6 | - | 3 | Ag ceramic particles | 1 | 15° | Equal to or less than 30 minutes | A | 26 | 3 | 2H | A | 10 |
| Example 3 | 32 | 32 | 22 | 10 | - | 3 | Ag ceramic particles | 1 | 22° | Equal to or less than 60 minutes | B | 18 | 3 | 2H | A | 10 |
| Example 4 | 30 | 30 | 22 | 14 | - | 3 | Ag ceramic particles | 1 | 29° | Equal to or less than 60 minutes | B | 15 | 2 | 3H | A | 18 |
| Example 5 | 30 | 30 | 22 | 13 | - | 3 | Ag ceramic particles | 2 | 27° | Equal to or less than 30 minutes | A | 30 | 2 | 3H | A | 20 |
| Example 6 | 30 | 30 | 22 | 12 | - | 3 | Ag ceramic particles | 3 | 24° | Equal to or less than 30 minutes | A | 40 | 2 | 3H | A | 21 |
| Example 7 | 30 | 30 | 22 | 10 | - | 3 | Ag ceramic particles | 5 | 22° | Equal to or less than 30 minutes | A | 80 | 2 | 3H | A | 22 |
| Example 8 | 25 | 25 | 22 | 24 | - | 3 | Ag ceramic particles | 5 | 48° | Equal to or less than 60 minutes | B | 20 | 1.8 | 3H | A | 24 |
| Example 9 | 34 | 34 | 22 | 2 | 4 | 3 | Ag ceramic particles | 1 | 15° | Equal to or less than 60 minutes | B | 16 | 5 | H | B | 1.40 |
| Example 10 | 32 | 32 | 22 | 2 | 8 | 3 | Ag ceramic particles | 1 | 13° | Equal to or less than 60 minutes | B | 18 | 8 | F | B | 1.35 |
| Example 11 | 37 | 37 | 22 | - | - | 3 | Ag ceramic particle + Ag particle dispersion | 1+1 | 8° | Equal to or less than 30 minutes | A | 27 | 3 | 2H | A | 10 |
| Example 12 | 32 | 32 | 22 | 2 | 8 | 3 | Ag ceramic particle + Ag particle dispersion | 1+1 | 13° | Equal to or less than 30 minutes | A | 25 | 8 | H | B | 1.40 |
| Example 13 | 10 | 10 | 22 | 54 | - | 3 | Ag ceramic particles | 1 | 80° | 2 hours | C | 12 | 1 | 2H | A | 8 |
| Example 14 | 30 | 30 | 22 | - | 14 | 3 | Ag ceramic particles | 1 | 10° | Equal to or less than 60 minutes | B | 18 | 12 | HB | C | 1.33 |
| Comparative Example 1 | - | - | 22 | 74 | - | 3 | Ag ceramic particles | 1 | 90° | Equal to or longer than 5 hours | E | 6 | 1 | 2H | A | 8 |
| Comparative Example 2 | Glass board with antibacterial coat (antibacterial film) described in Example 1 of JP2008-213206A | | | | | | Ag particle dispersion | 0.2 | 40° | 3 hours | D | 10 | 11 | HB | C | 1.30 |

**[Table 2]**

| Table 1 (1-2 ) | Hydrophilic monomer (1) M4004 (part by mass) | Hydrophilic monomer (2) M3150 (part by mass) | Cross-linking agent A-DPH (part by mass) | Monomer M420 (part by mass) | Polymerization initiator IRGACURE 184 (part by mass) | Silver-containing antibacterial agent | | Photocatalytic material | | Total amount Part by mass | Ratio of silver-containing antibacterial agent/photocatalytic material | Contact angle | Time taken for number of living bacteria to become equal to or less than limit of detection | Antibacterial properties | Amount of Ag ions (ng/cm²) | Water absorption rate (wt%) | Pencil hardness | Hard coat properties | Vickers hardness (HV) |
|---|---|---|---|---|---|---|---|---|---|---|---|---|---|---|---|---|---|---|---|
| | | | | | | Type | Added amount (part by mass) | Type | Added amount (part by mass) | | | | | | | (With respect to weight of antibacterial coat) | | | |
| Example 15 | 28 | 28 | 22 | 17 | 3 | Ag ceramic particle dispersion | 0.9 | TiO ₂ | 0.55 | 1.45 | 0.82 | 34° | Equal to or less than 30 minutes | A | 17 | 2 | 3H | A | 20 |
| Example 16 | 28 | 28 | 22 | 17 | 3 | Ag ceramic particle dispersion | 1.2 | TiO₂ | 0.4 | 1.6 | 1.50 | 35° | Equal to or less than 30 minutes | A | 19 | 2 | 3H | A | 20 |
| Example 17 | 28 | 28 | 22 | 17 | 3 | Ag ceramic particle dispersion | 0.6 | TiO₂ | 0.7 | 1.3 | 0.43 | 33° | Equal to or less than 30 minutes | A | 11 | 2 | 3H | A | 22 |
| Example 18 | 28 | 28 | 22 | 17 | 3 | Ag ceramic particle dispersion | 2.5 | TiO₂ | 1.25 | 3.75 | 1.00 | 30° | Equal to or less than 30 minutes | A | 40 | 2 | 3H | A | 21 |
| Example 19 | 28 | 28 | 22 | 17 | 3 | Ag ceramic particle dispersion | 1.5 | TiO ₂ | 0.25 | 1.75 | 3.00 | 30° | Equal to or less than 30 minutes | A | 26 | 2 | 3H | A | 20 |
| Example 20 | 28 | 28 | 22 | 17 | 3 | Ag ceramic particle dispersion | 0.12 | TiO ₂ | 0.19 | 0.31 | 0.32 | 30° | Equal to or less than 30 minutes | A | 3 | 2 | 3H | A | 20 |
| Example 21 | 28 | 28 | 22 | 17 | 3 | Ag ceramic particle dispersion | 0.75 | TiO ₂ | 0.375 | 1.125 | 1.00 | 28° | Equal to or less than 30 minutes | A | 15 | 2 | 3H | A | 20 |
| Example 22 | 28 | 28 | 22 | 17 | 3 | Ag ceramic particle dispersion | 2.3 | W O₃ | 0.35 | 2.65 | 3.29 | 30° | Equal to or less than 60 minutes | B | 38 | 2 | 3H | A | 21 |
| Example 23 | 28 | 28 | 22 | 17 | 3 | Ag ceramic particle dispersion | 0.64 | W O₃ | 0.18 | 0.82 | 1.78 | 25° | Equal to or less than 30 minutes | A | 13 | 2 | 3H | A | 22 |
| Example 24 | 28 | 28 | 22 | 17 | 3 | Ag ceramic particle dispersion | 1.56 | TiO₂ | 0.22 | 1.78 | 3.55 | 33° | Equal to or less than 60 minutes | B | 27 | 2 | 3H | A | 20 |
| Example 25 | 28 | 28 | 22 | 17 | 3 | Ag ceramic particle dispersion | 0.33 | TiO ₂ | 0.835 | 1.165 | 0.20 | 33° | Equal to or less than 60 minutes | B | 7 | 2 | 3H | A | 20 |
| Example 26 | 28 | 28 | 22 | 17 | 3 | Ag particle dispersion | 0.33 | TiO ₂ | 0.017 | 0.347 | 0.19 | 33° | Equal to or less than 60 minutes | B | 8 | 2 | 3H | A | 20 |
| Example 27 | 28 | 28 | 22 | 17 | 3 | Ag particle dispersion | 0.67 | TiO ₂ | 0.003 | 0.673 | 2.23 | 33° | Equal to or less than 30 minutes | A | 15 | 2 | 3H | A | 20 |
| Example 28 | 28 | 28 | 22 | 17 | 3 | Ag particle dispcrsion | 0.67 | W O₃ | 0.003 | 0.673 | 2.23 | 34° | Equal to or less than 30 minutes | A | 15 | 2 | 3H | A | 21 |

As is evident from Table 1, in Examples 1 to 14, a contact angle was equal to or less than 80°, and the time taken for the number of living bacteria to become equal to or less than a limit of detection was significantly shorter than in Comparative Example 1 in which a contact angle was greater than 80°. Furthermore, Examples 1 to 14 were evaluated to be A, B, or C in terms of antibacterial properties. In addition, in Examples 1 to 12 and 14, a contact angle was equal to or less than 60°, and an amount (Ag) of silver ions was equal to or greater than 15 ng/cm². In Examples 1 to 12 and 14, although a contact angle was equal to or less than 60°, the time taken for the number of living bacteria to become equal to or less than a limit of detection was significantly shorter than in Comparative Example 2 in which an amount of silver ions was less than 15 ng/cm². Examples 1 to 12 and 14 were evaluated to be A or B in terms of antibacterial properties.

As is evident from Table 1, in Examples 1 to 13 and 15 to 28 and Comparative Example 1 in which a water absorption rate was less than 10 wt%, unlike Comparative Example 2 in which a water absorption rate was equal to or greater than 10 wt%, a pencil hardness was 3H, 2H, H, or F, a Vickers hardness (HV) was equal to or greater than 1.35, and hard coat properties were evaluated to be A or B.

As is evident from Table 1, in Examples 11 and 12, in a case where silver particles are used together with silver ceramic particles as an antibacterial agent, antibacterial performance was obtained which was equal to or better than antibacterial performance obtained in a case where only silver ceramic particles are used.

From Examples 9, 10, 12, and 14, it was understood that if a hydrophilic polymer (PEG 200) is added as in JP2010-503737A and JP2008-213206A instead of a polymer cured and crosslinked by UV, antibacterial properties are not poor due to an increase of a contact angle, but hard coat properties deteriorate due to an increase of a water absorption rate. Furthermore, it was understood that if the amount of hydrophilic polymers is increased as in Example 14, hard coat properties are evaluated to be C and become poor. It was also understood that, while Comparative Example 2, that is evaluated to be C in terms of hard coat properties just like Example 14, has a Vickers hardness (HV) of 1.30, Example 14 has a Vickers hardness (HV) of 1.33 and has scratch resistance.

In Examples 15 to 28, a contact angle was equal to or less than 80°, and the time taken for the number of living bacteria to become equal to or less than a limit of detection was shortened under the condition of T/Da ≤ 300. Furthermore, Examples 15 to 28 were evaluated to be A or B in terms of antibacterial properties. In addition, through comparison between Examples 15 to 28, it was understood that in a case where a mass ratio of an antibacterial agent/a photocatalytic material is 0.3 to 3.0, better effects are obtained.

The effects of the present invention are clarified by the above results.

Furthermore, an outer surface of the mammography device was coated with a coating agent containing a composition for forming a hydrophilic processed portion containing an antibacterial agent as in Examples 1 to 28, and the coating agent was cured, thereby preparing a device with an antibacterial layer. As a result, it was confirmed that the same effects as shown in Tables 1 and 2 are obtained.

Hitherto, the instrument, the protective sheet, and the antibacterial film according to the present invention have been described based on various embodiments and examples, but the present invention is not limited to the embodiments and examples. It goes without saying that within a scope that does not depart from the gist of the present invention, the present invention may be embodied by being ameliorated in various ways or changed in terms of the design.

### Explanation of References

- 10, 70: portable radiographic imaging device
- 18, 72: housing
- 12, 74: radiation detector
- 13: control board
- 19, 86: irradiation surface
- 20, 87: hydrophilic processed portion
- 29: scintillator
- 30: TFT board
- 60: front panel
- 62: back panel
- 64: top panel
- 66: holding portion
- 76: body member
- 78, 80: cap member
- 82: battery mounting portion
- 84: connector connecting portion
- 90: mammography device
- 92: face guard
- 94: breast support
- 96: breast compression plate
- 100: radiographic diagnostic device
- 102: imaging board
- 104, 106: grip
- 110: display device
- 112: touch panel
- 112a: input surface
- 114: display unit
- 116: housing
- 118: conductive film
- 120: cover member
- 122: cable
- 124: flexible board
- 126: detection control portion
- 128: adhesive layer
- 130, 144: hydrophilic processed portion
- 132: contact object
- 140, 141: protective sheet
- 142: main sheet
- 146: pressure sensitive adhesive layer
- 148: release sheet
- 150, 150A, 150B: antibacterial substrate
- 152: substrate
- 154: hydrophilic portion
- 156: antibacterial film

## Claims

1. An instrument comprising:
a hydrophilic processed portion on at least a portion of an outer surface thereof,
wherein the hydrophilic processed portion contains a hydrophilic polymer and a silver-containing antibacterial agent,
a water contact angle of a surface of the hydrophilic processed portion is equal to or less than 60 °,
the hydrophilic processed portion further contains at least one kind of photocatalytic material containing a metal oxide, and
an amount of silver ions per unit area of the hydrophilic processed portion that is measured by the following extraction test is equal to or greater than 15 ng/cm²,
Extraction condition: A 1/500 normal nutrient broth medium specified in JIS Z 2801:2010 is used as an extractant; a temperature of the extractant is controlled within a range of 35 ± 1°C; the extractant is brought into contact with the surface of the hydrophilic processed portion for 1 hour; an amount of silver ions extracted into the extractant is measured; the obtained value is divided by a contact area between the surface of the hydrophilic processed portion and the extractant, thereby obtaining an amount of silver ions per unit area; and herein, a unit of the amount of silver ions is ng, a unit of the contact area is cm², and a unit of the amount of silver ions per unit area is ng/cm².

2. The instrument according to claim 1,
wherein the water contact angle of the surface of the hydrophilic processed portion is equal to or less than 60°, and
a water absorption rate of the hydrophilic processed portion is less than 10 wt%.

3. The instrument according to claim 1 or 2,
wherein the hydrophilic processed portion is formed using a coating agent containing at least a polymerizable compound, which has a hydrophilic group and two or more (meth)acryl groups, a cross-linking agent and the antibacterial agent, and
the hydrophilic group is a polyoxyethylene group.

4. The instrument according to any one of claims 1 to 3,
wherein the antibacterial agent contains at least either or both of silver-supporting ceramic particles and silver particles.

5. The instrument according to any one of claims 1 to 4 that is a touch panel,
wherein the hydrophilic processed portion is provided on the outer surface that a user contacts.

6. A protective sheet comprising:
a hydrophilic processed portion on at least a portion of an outer surface thereof,
wherein the hydrophilic processed portion contains a hydrophilic polymer and a silver-containing antibacterial agent,
a water contact angle of a surface of the hydrophilic processed portion is equal to or less than 60°,
the hydrophilic processed portion further contains at least one kind of photocatalytic material containing a metal oxide, and
an amount of silver ions per unit area of the hydrophilic processed portion that is measured by the following extraction test is equal to or greater than 15 ng/cm²,
Extraction condition: A 1/500 normal nutrient broth medium specified in JIS Z 2801:2010 is used as an extractant; a temperature of the extractant is controlled within a range of 35 ± 1°C; the extractant is brought into contact with the surface of the hydrophilic processed portion for 1 hour; an amount of silver ions extracted into the extractant is measured; the obtained value is divided by a contact area between the surface of the hydrophilic processed portion and the extractant, thereby obtaining an amount of silver ions per unit area; and herein, a unit of the amount of silver ions is ng, a unit of the contact area is cm², and a unit of the amount of silver ions per unit area is ng./cm².

7. The protective sheet according to claim 6,
wherein
a water absorption rate of the hydrophilic processed portion is less than 10 wt%.

8. The protective sheet according to claim 6 or 7,
wherein the hydrophilic processed portion is formed using a coating agent containing at least a polymerizable compound, which has a hydrophilic group and two or more (meth)acryl groups, a cross-linking agent and the antibacterial agent, and
the hydrophilic group is a polyoxyethylene group.

9. The protective sheet according to any one of claims 6 to 8,
wherein the antibacterial agent contains at least either or both of silver-supporting ceramic particles and silver particles.

10. An antibacterial film in which at least a portion is hydrophilic, comprising:
a hydrophilic portion that exhibits hydrophilicity and contains a hydrophilic polymer and a silver-containing antibacterial agent,
wherein a water contact angle of a surface of the hydrophilic portion is equal to or less than 60°,
the hydrophilic portion further contains at least one kind of photocatalytic material containing a metal oxide, and
an amount of silver ions per unit area of the hydrophilic portion that is measured by the following extraction test is equal to or greater than 15 ng/cm²,
Extraction condition: A 1/500 normal nutrient broth medium specified in JIS Z 2801:2010 is used as an extractant; a temperature of the extractant is controlled within a range of 35 ± 1°C; the extractant is brought into contact with the surface of the hydrophilic portion for 1 hour; an amount of silver ions extracted into the extractant is measured; the obtained value is divided by a contact area between the surface and the extractant, thereby obtaining an amount of silver ions per unit area; and herein, a unit of the amount of silver ions is ng, a unit of the contact area is cm², and a unit of the amount of silver ions per unit area is ng/cm²

11. The antibacterial film according to claim 10,
wherein the water contact angle of the surface of the hydrophilic portion is equal to or less than 60°, and
a water absorption rate of the hydrophilic portion is less than 10 wt%.

12. The antibacterial film according to claim 10 or 11,
wherein the hydrophilic portion is formed using a coating agent containing at least a polymerizable compound, which contains a hydrophilic group and two or more (meth)acryl groups, a cross-linking agent and the antibacterial agent, and
the hydrophilic group is a polyoxyethylene group.

13. The antibacterial film according to any one of claims 10 to 12,
wherein the antibacterial agent contains at least either or both of silver-supporting ceramic particles and silver particles.

## Patentansprüche

1. Instrument, umfassend:
einen hydrophil bearbeiteten Bereich auf zumindest einem Teil einer äußeren Oberfläche hiervon,
worin der hydrophil bearbeitete Bereich ein hydrophiles Polymer und ein silberhaltiges, antibakterielles Mittel enthält,
der Wasserkontaktwinkel einer Oberfläche des hydrophil bearbeiteten Bereichs gleich zu oder kleiner als 60° ist,
der hydrophil bearbeitete Bereich ferner zumindest eine Art von fotokatalytischem Material, enthaltend ein Metalloxid, enthält, und
die Menge an Silberionen je Flächeneinheit des hydrophil bearbeiteten Bereichs, die mit dem folgenden Extraktionstest gemessen wird, gleich zu oder größer als 15 ng/cm² ist,
Extraktionsbedingungen: als Extraktionsmittel wird ein 1/500 normales Nährmittel-Wachstumsmedium, spezifiziert in JIS Z 2801:2010, verwendet, die Temperatur des Extraktionsmittels wird innerhalb des Bereichs von 35 ± 1°C kontrolliert; das Extraktionsmittel wird mit der Oberfläche des hydrophil bearbeiteten Bereichs für 1 Stunde in Kontakt gebracht; die in das Extraktionsmittel extrahierte Menge an Silberionen wird gemessen; der erhaltene Wert wird durch die Kontaktfläche zwischen der Oberfläche des hydrophil bearbeiteten Bereichs und dem Extraktionsmittel geteilt, wodurch die Menge an Silberionen je Flächeneinheit erhalten wird; und hierin ist die Einheit der Menge der Silberionen ng, die Einheit der Kontaktfläche ist cm², und die Einheit der Menge der Silberionen je Flächeneinheit ist ng/cm².

2. Instrument gemäß Anspruch 1, worin der Wasserkontaktwinkel der Oberfläche des hydrophil bearbeiteten Bereichs gleich zu oder kleiner als 60° ist und
die Wasserabsorptionsrate des hydrophil bearbeiteten Bereichs weniger als 10 Gew.-% beträgt.

3. Instrument gemäß Anspruch 1 oder 2, worin der hydrophil bearbeitete Bereich gebildet ist unter Verwendung eines Beschichtungsmittels, enthaltend zumindest eine polymerisierbare Verbindung, die eine hydrophile Gruppe und zwei oder mehr (Meth)acrylgruppen enthält, ein Vernetzungsmittel und das antibakterielle Mittel, und
worin die hydrophile Gruppe eine Polyoxyethylengruppe ist.

4. Instrument gemäß irgendeinem der Ansprüche 1 bis 3, worin das antibakterielle Mittel zumindest eines oder beides von Silber-tragenden Keramikpartikeln und Silberpartikeln enthält.

5. Instrument gemäß irgendeinem der Ansprüche 1 bis 4, welches ein Touch-Panel ist, worin der hydrophil bearbeitete Bereich auf der äußeren Oberfläche vorgesehen ist, die ein Benutzer berührt.

6. Schutzfolie, umfassend:
einen hydrophil bearbeiteten Bereich auf zumindest einem Bereich einer äußeren Oberfläche hiervon,
worin der hydrophil bearbeitete Bereich ein hydrophiles Polymer und ein silberhaltiges antibakterielles Mittel enthält,
der Wasserkontaktwinkel einer Oberfläche des hydrophil bearbeiteten Bereichs gleich zu oder kleiner als 60° ist,
der hydrophil bearbeitete Bereich ferner zumindest eine Art von fotokatalytischem Material, enthaltend ein Metalloxid, enthält, und
die Menge an Silberionen je Flächeneinheit des hydrophil bearbeiteten Bereichs, die durch den folgenden Extraktionstest gemessen wird, gleich zu oder größer als 15 ng/cm² ist
Extraktionsbedingung: als Extraktionsmittel wird ein 1/500 normales Nährmittel-Wachstumsmedium, spezifiziert in JIS Z 2801:2010, verwendet, die Temperatur des Extraktionsmittels wird innerhalb des Bereichs von 35 ± 1°C kontrolliert; das Extraktionsmittel wird mit der Oberfläche des hydrophil bearbeiteten Bereichs für 1 Stunde in Kontakt gebracht; die in das Extraktionsmittel extrahierte Menge an Silberionen wird gemessen; der erhaltene Wert wird durch die Kontaktfläche zwischen der Oberfläche des hydrophil bearbeiteten Bereichs und dem Extraktionsmittel geteilt, wodurch die Menge an Silberionen je Flächeneinheit erhalten wird; und hierin ist die Einheit der Menge der Silberionen ng, die Einheit der Kontaktfläche ist cm², und die Einheit der Menge der Silberionen je Flächeneinheit ist ng/cm².

7. Schutzfolie gemäß Anspruch 6, worin der Wasserkontaktwinkel der Oberfläche des hydrophil bearbeiteten Bereichs gleich zu oder kleiner als 60° ist und
die Wasserabsorptionsrate des hydrophil bearbeiteten Bereichs weniger als 10 Gew.-% beträgt.

8. Schutzfolie gemäß Anspruch 6 oder 7, worin der hydrophil bearbeitete Bereich gebildet ist unter Verwendung eines Beschichtungsmittels, enthaltend zumindest eine polymerisierbare Verbindung, die eine hydrophile Gruppe und zwei oder mehr (Meth)acrylgruppen enthält, ein Vernetzungsmittel und das antibakterielle Mittel, und
worin die hydrophile Gruppe eine Polyoxyethylengruppe ist.

9. Schutzfolie gemäß irgendeinem der Ansprüche 6 bis 8, worin das antibakterielle Mittel zumindest eines oder beides von Silber-tragenden Keramikpartikeln und Silberpartikeln enthält.

10. Antibakterieller Film, bei dem zumindest ein Teil hydrophil ist, umfassend:
einen hydrophilen Bereich, der Hydrophilität zeigt und ein hydrophiles Polymer und ein silberhaltiges antibakterielles Mittel enthält,
worin der Wasserkontaktwinkel einer Oberfläche des hydrophilen Bereichs gleich zu oder kleiner als 60° ist, der hydrophile Bereich ferner zumindest eine Art von fotokatalytischem Material, enthaltend ein Metalloxid, enthält, und
die Menge an Silberionen je Flächeneinheit des hydrophilen Bereichs, die gemessen wird mit dem folgenden Extraktionstest, gleich zu oder größer als 15 ng/cm² ist,
Extraktionsbedingungen: als Extraktionsmittel wird ein 1/500 normales Nährmittel-Wachstumsmedium, spezifiziert in JIS Z 2801:2010, verwendet, die Temperatur des Extraktionsmittels wird innerhalb des Bereichs von 35 ± 1°C kontrolliert; das Extraktionsmittel wird mit der Oberfläche des hydrophilen Bereichs für 1 Stunde in Kontakt gebracht; die in das Extraktionsmittel extrahierte Menge an Silberionen wird gemessen; der erhaltene Wert wird durch die Kontaktfläche zwischen der Oberfläche und dem Extraktionsmittel geteilt, wodurch die Menge an Silberionen je Flächeneinheit erhalten wird; und hierin ist die Einheit der Menge der Silberionen ng, die Einheit der Kontaktfläche ist cm², und die Einheit der Menge der Silberionen je Flächeneinheit ist ng/cm².

11. Antibakterieller Film gemäß Anspruch 10, worin der Wasserkontaktwinkel der Oberfläche des hydrophilen Bereichs gleich zu oder kleiner als 60° ist und
die Wasserabsorptionsrate des hydrophilen Bereichs weniger als 10 Gew.-% beträgt.

12. Antibakterieller Film gemäß Anspruch 10 oder 11, worin der hydrophile Bereich gebildet ist unter Verwendung eines Beschichtungsmittels, enthaltend zumindest eine polymerisierbare Verbindung, die eine hydrophile Gruppe und zwei oder mehr (Meth)acrylgruppen enthält, ein Vernetzungsmittel und das antibakterielle Mittel, und
worin die hydrophile Gruppe eine Polyoxyethylengruppe ist.

13. Antibakterieller Film gemäß irgendeinem der Ansprüche 10 bis 12, worin das antibakterielle Mittel zumindest eines oder beides von Silber-tragenden Keramikpartikeln und Silberpartikeln enthält.

## Revendications

1. Dispositif comprenant :
une partie rendue hydrophile sur au moins une partie d'une surface extérieure de celui-ci,
dans lequel la partie rendue hydrophile contient un polymère hydrophile et un agent antibactérien contenant de l'argent,
un angle de contact avec l'eau d'une surface de la partie rendue hydrophile est inférieur ou égal à 60 °,
la partie rendue hydrophile contient en outre au moins un type de matériau photocatalytique contenant un oxyde métallique, et
une quantité d'ions argent par unité de surface de la partie rendue hydrophile qui est mesurée par le test d'extraction suivant est supérieure ou égale à 15 ng/cm²,
condition d'extraction : un bouillon nutritif normal à 1/500 spécifié dans la norme JIS Z 2801:2010 est utilisé comme agent d'extraction; une température de l'agent d'extraction est contrôlée dans une plage de 35 ± 1° C ; l'agent d'extraction est mis en contact avec la surface de la partie rendue hydrophile pendant 1 heure ; une quantité d'ions argent extraits dans l'agent d'extraction est mesurée ; la valeur obtenue est divisée par une surface de contact entre la surface de la partie rendue hydrophile et l'agent d'extraction, obtenant ainsi une quantité d'ions argent par unité de surface ; et dans le cas présent, une unité de la quantité d'ions argent est en ng, une unité de la surface de contact est en cm², et une unité de la quantité d'ions argent par unité de surface est en ng/cm².

2. Dispositif selon la revendication 1,
dans lequel l'angle de contact avec l'eau de la surface de la partie rendue hydrophile est inférieur ou égal à 60°, et
un taux d'absorption d'eau de la partie rendue hydrophile est inférieur à 10 % en poids.

3. Dispositif selon la revendication 1 ou 2,
dans lequel la partie rendue hydrophile est formée en utilisant un agent de revêtement contenant au moins un composé polymérisable qui présente un groupe hydrophile et deux ou plusieurs groupes (méth)acryle, un agent de réticulation et l'agent antibactérien, et
le groupe hydrophile est un groupe polyoxyéthylène.

4. Dispositif selon l'une quelconque des revendications 1 à 3,
dans lequel l'agent antibactérien contient au moins l'un ou l'autre ou les deux parmi des particules de céramique supportant l'argent et des particules d'argent.

5. Dispositif selon l'une quelconque des revendications 1 à 4 qui est un écran tactile,
dans lequel la partie rendue hydrophile est fournie sur la surface extérieure avec laquelle un utilisateur entre en contact.

6. Feuille protectrice comprenant :
une partie rendue hydrophile sur au moins une partie d'une surface extérieure de celle-ci,
dans laquelle la partie rendue hydrophile contient un polymère hydrophile et un agent antibactérien contenant de l'argent,
un angle de contact avec l'eau d'une surface de la partie rendue hydrophile est inférieur ou égal à 60°,
la partie rendue hydrophile contient en outre au moins un type de matériau photocatalytique contenant un oxyde métallique, et
une quantité d'ions argent par unité de surface de la partie rendue hydrophile qui est mesurée par le test d'extraction suivant est supérieure ou égale à 15 ng/cm²,
condition d'extraction : un bouillon nutritif normal à 1/500 spécifié dans la norme JIS Z 2801:2010 est utilisé comme agent d'extraction; une température de l'agent d'extraction est contrôlée dans une plage de 35 ± 1° C ; l'agent d'extraction est mis en contact avec la surface de la partie rendue hydrophile pendant 1 heure ; une quantité d'ions argent extraits dans l'agent d'extraction est mesurée ; la valeur obtenue est divisée par une surface de contact entre la surface de la partie rendue hydrophile et l'agent d'extraction, obtenant ainsi une quantité d'ions argent par unité de surface ; et dans le cas présent, une unité de la quantité d'ions argent est en ng, une unité de la surface de contact est en cm², et une unité de la quantité d'ions argent par unité de surface est en ng/cm².

7. Feuille protectrice selon la revendication 6,
dans laquelle
un taux d'absorption d'eau de la partie rendue hydrophile est inférieur à 10 % en poids.

8. Feuille protectrice selon la revendication 6 ou 7,
dans laquelle la partie rendue hydrophile est formée en utilisant un agent de revêtement contenant au moins un composé polymérisable qui présente un groupe hydrophile et deux ou plusieurs groupes (méth)acryle, un agent de réticulation et l'agent antibactérien, et
le groupe hydrophile est un groupe polyoxyéthylène.

9. Feuille protectrice selon l'une quelconque des revendications 6 à 8,
dans laquelle l'agent antibactérien contient au moins l'un ou l'autre ou les deux parmi des particules de céramique supportant l'argent et des particules d'argent.

10. Film antibactérien dans lequel au moins une partie est hydrophile, comprenant :
une partie hydrophile qui présente une hydrophilie et contient un polymère hydrophile et un agent antibactérien contenant de l'argent,
dans lequel un angle de contact avec l'eau d'une surface de la partie rendue hydrophile est inférieur ou égal à 60°,
la partie rendue hydrophile contient en outre au moins un type de matériau photocatalytique contenant un oxyde métallique, et
une quantité d'ions argent par unité de surface de la partie rendue hydrophile qui est mesurée par le test d'extraction suivant est supérieure ou égale à 15 ng/cm²,
condition d'extraction : un bouillon nutritif normal à 1/500 spécifié dans la norme JIS Z 2801:2010 est utilisé comme agent d'extraction; une température de l'agent d'extraction est contrôlée dans une plage de 35 ± 1° C ; l'agent d'extraction est mis en contact avec la surface de la partie rendue hydrophile pendant 1 heure ; une quantité d'ions argent extraits dans l'agent d'extraction est mesurée ; la valeur obtenue est divisée par une surface de contact entre la surface et l'agent d'extraction, obtenant ainsi une quantité d'ions argent par unité de surface ; et dans le cas présent, une unité de la quantité d'ions argent est en ng, une unité de la surface de contact est en cm², et une unité de la quantité d'ions argent par unité de surface est en ng/cm²

11. Film antibactérien selon la revendication 10,
dans lequel l'angle de contact avec l'eau de la surface de la partie rendue hydrophile est inférieur ou égal à 60°, et
un taux d'absorption d'eau de la partie rendue hydrophile est inférieur à 10 % en poids.

12. Film antibactérien selon la revendication 10 ou 11,
dans lequel la partie rendue hydrophile est formée en utilisant un agent de revêtement contenant au moins un composé polymérisable qui contient un groupe hydrophile et deux ou plusieurs groupes (méth)acryle, un agent de réticulation et l'agent antibactérien, et
le groupe hydrophile est un groupe polyoxyéthylène.

13. Film antibactérien selon l'une quelconque des revendications 10 à 12,
dans lequel l'agent antibactérien contient au moins l'un ou l'autre ou les deux parmi des particules de céramique supportant l'argent et des particules d'argent.
